# EUROPÄISCHE PATENTANMELDUNG

(11) **EP 4 361 478 A1**
(43) Veröffentlichungstag der Anmeldung: **01.05.2024**
(21) Anmeldenummer: 23199831.1
(22) Anmeldetag: 26.09.2023
(51) Int. Cl.: F16K 27/00, A61L 2/10, F16K 27/02, F16K 27/10, F16K 31/12

(54) **STELLVENTIL MIT EINER STERILISIERUNGSEINHEIT**

(30) Priorität: 24.10.2022 DE 102022127996
(71) Anmelder: Samson Aktiengesellschaft, 60314 Frankfurt am Main (DE)
(72) Erfinder: Dr. Steckenreiter, Thomas, 63322 Rödermark (DE); Soulas, Bruno, 69003 Lyon (FR); Lachenal-Chevallet, Michael, 69340 Francheville (FR); Rutsch, Uwe, 60314 Frankfurt am Main (DE)
(74) Vertreter: noventive Patentanwaltsgesellschaft mbH

(57) **Zusammenfassung**

Die Erfindung geht aus von einem Stellventil (10a; 10b; 10c) für eine Prozessanlage, insbesondere mit hohen Reinlichkeitsanforderungen, beispielsweise in der Lebensmittel- und/oder der Pharmaindustrie, mit einem Ventilgehäuse (12a; 12b; 12c), das zumindest eine Drosselstelle (14a; 14b; 14c) für ein Prozessmedium ausbildet, mit einem Ventilglied (16a; 16b; 16c), mit einer Ventilstange (18a; 18b; 18c) und mit einer Antriebseinheit (20a; 20b; 20c), die dazu vorgesehen ist, das Ventilglied (16a; 16b; 16c) über die Ventilstange (18a; 18b; 18c) in einem Bereich der Drosselstelle (14a; 14b; 14c) zu bewegen.

Es wird vorgeschlagen, dass das Stellventil (10a; 10b; 10c) eine Sterilisierungseinheit (36a; 36b; 36c) zu einem Sterilisieren von Innenflächen (40a; 40b; 40c) des Stellventils (10a; 10b; 10c), insbesondere des Ventilgehäuses (12a; 12b; 12c), und/oder des Prozessmediums, wobei die Sterilisierungseinheit (36a; 36b; 36c) zumindest eine UV-Lichtquelle (38a, 38a"; 38b; 38b', 38b"; 38c) umfasst.

## Beschreibung

### Stand der Technik

In Prozessanlagen, beispielsweise für die Lebensmittel- und Pharmaindustrie, werden oft hohe Anforderungen an die Reinlichkeit der Geräte gestellt, um ungewollte Verunreinigungen oder Wechselwirkungen der Prozessmedien zu verhindern. Stellventile für derartige Prozessanlagen müssen demnach dazu ausgelegt sein, Verunreinigungen und ein Entstehen von Keimen etc. im Prozessmedium zu verhindern. Dies ist besonders in Stellventilen relevant, da dort unterschiedliche Strömungsverhalten für das Prozessmedium auftreten können. Um ein Entstehen von Bakterien oder Keimen zu verhindern, ist es von Vorteil, das Prozessmedium und/oder Oberflächen in das Prozessmedium führenden Kammern zu sterilisieren.

Im Stand der Technik erfolgt eine Sterilisierung eines Bereichs einer Prozessanlage, welcher ein Stellventil umfasst, nach einer Montage des Stellventils, bevor Prozessmedium darin geführt wird. Zur Sterilisierung von Oberflächen und Medien eignet sich UV-Strahlung, insbesondere im UVc-Bereich des elektromagnetischen Spektrums. In den letzten Jahren wurden zur Sterilisierung von Oberflächen und Medien als LEDs ausgebildete Lichtquellen entwickelt, welche insbesondere UV-Strahlung im UVc-Bereich erzeugen. Diese UVc-LEDs eignen sich unter bestimmten Bedingungen zur Sterilisierung von Oberflächen und Medien, sind kompakt und energieeffizient.

Ausgehend vom Stand der Technik ist die Aufgabe der hier beschriebenen Erfindung ein Stellventil mit einer Sterilisierungseinheit zur Sterilisierung innerhalb von Prozessmedium führenden Volumen bereit zu stellen.

### Vorteile der Erfindung

Die Erfindung geht aus von einem Stellventil für eine Prozessanlage, insbesondere mit hohen Reinlichkeitsanforderungen, beispielsweise in der Lebensmittel- und/oder der Pharmaindustrie, mit einem Ventilgehäuse, das zumindest eine Drosselstelle für ein Prozessmedium ausbildet, mit einem Ventilglied, mit einer Ventilstange und mit einer Antriebseinheit, die dazu vorgesehen ist, das Ventilglied über die Ventilstange in einem Bereich der Drosselstelle zu bewegen.

Es wird vorgeschlagen, dass das Stellventil eine Sterilisierungseinheit zu einem Sterilisieren von Innenflächen des Stellventils, insbesondere des Ventilgehäuses, und/oder des Prozessmediums aufweist, wobei die Sterilisierungseinheit zumindest eine UV-Lichtquelle umfasst. Die Sterilisierungseinheit ist vorzugsweise dazu vorgesehen, die Innenflächen des Stellventils, insbesondere des Ventilgehäuses, und/oder das Prozessmedium von lebenden Mikroorganismen, wie Keime usw., und deren Ruhestadien, wie Sporen usw., zu befreien, was insbesondere im Folgenden als sterilisieren bezeichnet wird. Vorzugsweise ist die Sterilisierungseinheit dazu vorgesehen, mit Prozessmedium in zumindest einem Betriebszustand in Kontakt kommende Innenflächen des Stellventils, insbesondere des Ventilgehäuses, zu sterilisieren. Bevorzugt ist die Sterilisierungseinheit zu einer Sterilisierung der Innenflächen und/oder des Prozessmediums mittels elektromagnetischer Strahlung, insbesondere im UV-Bereich des elektromagnetischen Spektrums (im Folgenden auch UV-Strahlung genannt), vorgesehen. Insbesondere ist die Sterilisierungseinheit zu einer im Wesentlichen kontaktfreien Sterilisierung der Innenflächen und/oder des Prozessmediums vorgesehen. Die Sterilisierungseinheit, insbesondere die zumindest eine UV-Lichtquelle, ist vorzugsweise zumindest teilweise innerhalb des Ventilgehäuses angeordnet. Vorzugsweise umfasst die Sterilisierungseinheit eine Versorgungseinheit, wie eine Spannungsquelle, ein Netzanschluss o.dgl., die zu einer Versorgung der zumindest einen UV-Lichtquelle vorgesehen ist. Die Versorgungseinheit ist insbesondere außerhalb des Ventilgehäuses angeordnet. Bevorzugt ist die Sterilisierungseinheit, insbesondere die zumindest eine UV-Lichtquelle und/oder die Versorgungseinheit, an dem Ventilgehäuse angeordnet, insbesondere befestigt. Vorzugsweise ist die Sterilisierungseinheit, insbesondere die UV-Lichtquelle, zu einer Sterilisierung von zumindest einer oder mehrerer Innenflächen des Ventilgehäuses, zumindest einer Innenfläche eines Ventilsitzes des Stellventils, zumindest einer Innenfläche des Ventilglieds, zumindest einer Innenfläche von Verbindungselementen des Stellventils und/oder von Innenflächen anderer Bauteile des Stellventils vorgesehen.

Insbesondere soll unter einer "UV-Lichtquelle" ein Gerät verstanden werden, dass Strahlung im UV-Bereich des elektromagnetischen Spektrums erzeugt, und vorzugsweise zu einer Sterilisierung von Oberflächen und/oder Medien, insbesondere des Prozessmediums, geeignet ist. Insbesondere ist die zumindest eine UV-Lichtquelle als ein Sterilisierungselement der Sterilisierungseinheit ausgebildet. Es ist denkbar, dass die Sterilisierungseinheit lediglich eine UV-Lichtquelle oder mehr als eine UV-Lichtquelle umfasst, insbesondere auch wenn im Folgenden nur eine UV-Lichtquelle genannt ist. Die UV-Lichtquelle ist vorzugsweise dazu vorgesehen, zur Sterilisierung der Innenflächen und/oder des Prozessmediums UV-Strahlung in Richtung der Innenflächen bzw. in das Prozessmedium auszugeben.

Vorzugsweise umschließt das Ventilgehäuse ein Volumen, welches bevorzugt als ein Fluidkanal ausgebildet ist und vorzugsweise zu einem Führen von Prozessmedium vorgesehen ist.

Insbesondere ist die UV-Lichtquelle zumindest teilweise in oder angrenzend an den Fluidkanal angeordnet. Das Ventilgehäuse weist vorzugsweise zumindest eine Eintrittsöffnung und zumindest eine Austrittsöffnung auf. Bevorzugt ist das Ventilgehäuse an der Eintrittsöffnung und der Austrittsöffnung jeweils offen ausgebildet. Bevorzugt erstreckt sich der Fluidkanal innerhalb des Ventilgehäuses von der Eintrittsöffnung durch die Drosselstelle hindurch bis zur Austrittsöffnung.

Es sind verschiedene Ausgestaltungen des Stellventils denkbar, insbesondere unabhängig von einer Betriebsart und/oder einer Bewegungsart des Ventilglieds des Stellventils. Bevorzugt ist das Ventilglied des Stellventils über die Ventilstange mit der Antriebseinheit des Stellventils verbunden. Vorzugsweise ist die Ventilstange dazu vorgesehen, das Ventilglied bei einem Betrieb des Stellventils zu bewegen, wobei insbesondere das Ventilglied dazu vorgesehen ist, die Drosselstelle in zumindest einem Betriebszustand für das Prozessmedium zumindest teilweise und/oder vollständig zu verschließen. Bevorzugt ist die Sterilisierungseinheit, insbesondere die UV-Lichtquelle, beabstandet von dem das Prozessmedium führenden Fluidkanal des Stellventils angeordnet, insbesondere über ein Bauteil, wie einem Fensterelement o.dgl., von dem Prozessmedium bzw. dem Fluidkanal getrennt. Insbesondere ist die Sterilisierungsvorrichtung für eine Verwendung mit einem als Sicherheitsarmatur oder als Regelarmatur ausgebildeten Stellventil geeignet.

Durch die erfindungsgemäße Ausgestaltung des Stellventils kann eine vorteilhaft hohe Reinlichkeit innerhalb des Stellventils erreicht werden. Es kann eine vorteilhaft hohe Funktionalität des Stellventils ermöglicht werden. Bevorzugt kann eine Verwendung des Stellventils in Prozessanlagen zur Herstellung von Lebensmitteln, Arzneimitteln oder anderen Produkten, die eine hohe Reinlichkeit bei der Herstellung benötigen, ermöglicht werden. Insbesondere können weitere Schritte zu einer Sterilisierung eines Innenraums des Stellventils, beispielsweise nach einer Montage des Stellventils, vorteilhaft entfallen. Beispielsweise können Schweißstellen, Drosselstellen und/oder lokale Vertiefungen in einem Prozessmedium führenden Innenraum vorteilhaft einfach sterilisiert werden.

Des Weiteren wird vorgeschlagen, dass die UV-Lichtquelle als eine LED, insbesondere als eine UVc-LED, ausgebildet ist und insbesondere dazu vorgesehen ist, Strahlung im UVc-Bereich des elektromagnetischen Spektrums zu erzeugen. Es kann eine vorteilhaft kompakte Ausgestaltung der Sterilisierungseinheit ermöglicht werden. Dadurch kann eine vorteilhaft flexible und zur Sterilisierung vorteilhaft effektive Anordnung der UV-Lichtquelle ermöglicht werden. Es kann eine vorteilhaft hohe Energieeffizienz ermöglicht werden. Es kann eine vorteilhaft einfache und kostengünstige Versorgung der Sterilisierungseinheit erreicht werden. Durch eine Verwendung von UVc-Strahlung kann ein vorteilhaft schnelles und effizientes Sterilisieren ermöglicht werden. Besonders bevorzugt ist die UV-Lichtquelle dazu vorgesehen, UV-Strahlung im UVc-Bereich des elektromagnetischen Spektrums, insbesondere mit einer Wellenlänge zwischen 100 nm und 280 nm, vorzugsweise zwischen 200 nm und 270 nm und besonders bevorzugt zwischen 250 nm und 260 nm, zu erzeugen. Die als UV-LED ausgebildete UV-Lichtquelle weist vorzugsweise eine maximale Längs- bzw. Quererstreckung von höchstens 1 cm, bevorzugt höchstens 0,7 cm und besonders bevorzugt höchstens 0,5 cm, auf.

Alternativ ist denkbar, dass die UV-Lichtquelle als eine andere, insbesondere von einer LED verschiedene, Art von Lichtquelle ausgebildet ist, beispielsweise als eine Leuchtstofflampe o.dgl.

Ferner wird vorgeschlagen, dass die UV-Lichtquelle dazu vorgesehen ist, in zumindest einem Betriebszustand gepulst betrieben zu werden. Es kann eine vorteilhaft effektive und energiesowie zeiteffiziente Sterilisierung der Innenflächen und/oder des Prozessmediums ermöglicht werden. Im Vergleich zu einer kontinuierlichen Beleuchtung kann durch einen gepulsten Betrieb der UV-Lichtquelle bei gleichem Energiebedarf eine vorteilhaft schnelle Sterilisierung der Innenflächen und/oder des Prozessmediums erreicht werden. Vorzugsweise ist die UV-Lichtquelle dazu vorgesehen, in periodisch oder in sich verändernden zeitlichen Abständen Strahlung zur Sterilisierung auszugeben. Bevorzugt ist die UV-Lichtquelle dazu vorgesehen, Strahlung jeweils in Pulsen auszugeben. Insbesondere ist die UV-Lichtquelle dazu vorgesehen, Strahlung, insbesondere die UV-Strahlung, mit einer Pulsdauer von höchstens 100 ms, vorzugsweise höchstens 10 ms und besonders bevorzugt höchstens 1 ms, auszugeben. Bevorzugt beträgt eine Frequenz der UV-Lichtquelle, insbesondere der Pulse der UV-Lichtquelle, mindestens 0,5 Hz, vorzugsweise mindestens 1 Hz und besonders bevorzugt mindestens 2 Hz. Die UV-Lichtquelle weist eine Leistungsaufnahme zwischen 40 W und 800 W, vorzugsweise zwischen 40 W und 600 W und besonders bevorzugt zwischen 40 W und 200 W, auf. Beispielsweise ist die gepulste UV-Lichtquelle als ein mit Xenon-Gas gefülltes Volumen ausgebildet, wobei ein elektrischer Puls zur Erzeugung der UV-Strahlung vorgesehen ist. Es sind aber auch andere Ausgestaltungen der UV-Lichtquelle denkbar. Vorzugsweise ist die Sterilisationseinheit dazu vorgesehen, die zumindest eine UV-Lichtquelle in Abhängigkeit von zumindest einem Bewegungsparameter des Stellventils, insbesondere des Ventilglieds, und/oder von zumindest einem Prozessparameter des Prozessmediums im bzw. am Stellventils zu aktivieren. Vorzugsweise ist Sterilisationseinheit, insbesondere die UV-Lichtquelle, dazu vorgesehen, die Pulsdauer und/oder die Frequenz der UV-Lichtquelle in Abhängigkeit von dem Bewegungsparameter und/oder dem Prozessparameter anzupassen. Unter einem "Bewegungsparameter" des Stellventils/des Ventilglieds soll insbesondere ein Parameter verstanden werden, der eine Bewegung des Stellventils/des Ventilglieds, insbesondere bei einem Einstellen einer Ventilstellung, beschreibt. Beispielsweise ist der zumindest eine Bewegungsparameter als eine/ein, insbesondere maximale oder mittlere, Hublänge oder Drehwinkel des Ventilglieds, als eine, insbesondere maximale oder mittlere, Hub- oder Drehfrequenz des Ventilglieds, als eine Aktivierungsfrequenz einer Antriebseinheit des Stellventils o.dgl. ausgebildet. Unter einem "Prozessparameter" des Prozessmediums soll insbesondere ein Parameter verstanden werden, der eine die Sterilisation beeinflussende Eigenschaft des Prozessmediums beschreibt. Beispielsweise ist der zumindest eine Prozessparameter als eine Temperatur, als eine Dichte, als ein Durchfluss des Prozessmediums o.dgl. ausgebildet.

Zudem wird vorgeschlagen, dass das Ventilgehäuse zumindest einen, insbesondere den vorher genannten, Fluidkanal für das Prozessmedium ausbildet, der zumindest teilweise von der Drosselstelle begrenzt ist, wobei das Ventilgehäuse zumindest eine dem Fluidkanal zugewandte Innenfläche aufweist, wobei die zumindest eine UV-Lichtquelle an der Innenfläche des Ventilgehäuses angeordnet ist. Es kann eine vorteilhafte Anordnung der UV-Lichtquelle zur Sterilisierung innerhalb des Ventilgehäuses erreicht werden. Insbesondere kann eine vorteilhaft stabile Befestigung der UV-Lichtquelle innerhalb des Ventilgehäuses ermöglicht werden. Vorzugsweise ist die zumindest eine UV-Lichtquelle über die Innenfläche an dem Ventilgehäuse befestigt, beispielsweise über Befestigungsmittel des Stellventils. Der Fluidkanal erstreckt sich insbesondere von der Eintrittsöffnung über die Drosselstelle bis zur Austrittsöffnung. Es ist denkbar, dass die zumindest eine UV-Lichtquelle bzw. zumindest eine UV-Lichtquelle der Sterilisierungseinheit in einem Bereich der Drosselstelle, in einem Bereich der Eintrittsöffnung und/oder in einem Bereich der Austrittsöffnung angeordnet ist. Alternativ sind auch andere Ausgestaltungen der Sterilisierungseinheit hinsichtlich einer Anordnung der UV-Lichtquelle(n) denkbar. Bevorzugt ist die zumindest eine UV-Lichtquelle derart angeordnet, dass eine Hauptabstrahlrichtung der UV-Lichtquelle von der Innenfläche des Ventilgehäuses in Richtung des Fluidkanals ausgerichtet ist. Unter einer "Hauptabstrahlrichtung" einer Lichtquelle, insbesondere der UV-Lichtquelle, soll insbesondere eine Richtung verstanden werden, die sich von der Lichtquelle aus entlang einer Achse durch die Lichtquelle erstreckt, auf welcher ein Maximum einer Intensitätsverteilung von durch die Lichtquelle erzeugter Strahlung angeordnet ist. Bevorzugt ist die UV-Lichtquelle derart angeordnet, dass die Hauptabstrahlrichtung der UV-Lichtquelle den Fluidkanal, vorzugsweise einen Bereich um eine Mittelachse der Drosselstelle, der Eintrittsöffnung bzw. der Austrittsöffnung, die sich insbesondere zumindest teilweise innerhalb des Fluidkanals erstreckt, schneidet. Es ist denkbar, dass die UV-Lichtquelle derart angeordnet ist, dass die Hauptabstrahlrichtung einen Winkel von im Wesentlichen 90° zu einer die UV-Lichtquelle tragenden Innenfläche aufspannt. Alternativ oder zusätzlich ist denkbar, dass die UV-Lichtquelle derart angeordnet und/oder ausgebildet ist, dass die Hauptabstrahlrichtung der UV-Lichtquelle in einem von 90° verschiedenen Winkel zur jeweiligen Innenfläche, auf der die UV-Lichtquelle angeordnet ist, ausgerichtet ist. Alternativ oder zusätzlich ist denkbar, dass die UV-Lichtquelle auf einer relativ zu einer den Fluidkanal begrenzenden und von der UV-Lichtquelle beabstandeten Innenfläche des Ventilgehäuses, die sich um die UV-Lichtquelle bzw. um die die UV-Lichtquelle tragende Innenfläche herum erstreckt, geneigt ausgebildeten Innenfläche angeordnet ist.

Ferner wird vorgeschlagen, dass das Ventilgehäuse zumindest einen Fluidkanal für das Prozessmedium ausbildet, der zumindest teilweise von der Drosselstelle begrenzt ist, wobei die zumindest eine UV-Lichtquelle zumindest teilweise, insbesondere größtenteils oder vorzugsweise im Wesentlichen vollständig, innerhalb des Fluidkanals angeordnet ist. Es kann eine vorteilhaft direkte und effiziente Bestrahlung des Prozessmediums durch die UV-Lichtquelle ermöglicht werden. Vorzugsweise umfasst das Ventilgehäuse eine, insbesondere im Wesentlichen stetig ausgebildete, Führungsfläche, welche den Fluidkanal zumindest größtenteils begrenzt. Es ist denkbar, dass die UV-Lichtquelle zumindest teilweise über die Führungsfläche hinaus in den Fluidkanal hineinragt. Alternativ ist denkbar, dass die zumindest eine UV-Lichtquelle, insbesondere über die Führungsfläche hinaus, größtenteils oder im Wesentlichen vollständig innerhalb des Fluidkanals angeordnet ist. Insbesondere ist denkbar, dass die Sterilisierungseinheit ein Trägerelement, wie eine Stütze, einen Arm o.dgl., aufweist, der sich, insbesondere über die Führungsfläche hinaus, in den Fluidkanal erstreckt, wobei vorzugsweise die zumindest eine UV-Lichtquelle bzw. eine UV-Lichtquelle der Sterilisierungseinheit an dem Trägerelement angeordnet ist. Vorzugsweise ist das Trägerelement dazu vorgesehen, die UV-Lichtquelle innerhalb des Fluidkanals anzuordnen und gegen eine vom Prozessmedium auf die UV-Lichtquelle wirkende Kraft abzustützen.

Des Weiteren wird vorgeschlagen, dass das Ventilgehäuse an einer den Fluidkanal begrenzenden Innenwand eine Ausnehmung begrenzt, wobei die zumindest eine UV-Lichtquelle zumindest teilweise, insbesondere im Wesentlichen vollständig, innerhalb der Ausnehmung angeordnet ist. Es kann eine vorteilhaft strömungsresistente Anordnung der UV-Lichtquelle erreicht werden. Insbesondere kann ein ungewolltes Angreifen von über das Prozessmedium bewirkte Kräfte an der UV-Lichtquelle vorteilhaft verhindert werden. Dadurch kann eine vorteilhaft hohe Lebensdauer der UV-Lichtquelle bei einem Betrieb mit Prozessmedium erreicht werden. Die Ausnehmung grenzt vorzugsweise an die, insbesondere oben genannte, Innenfläche des Ventilgehäuses. Bevorzugt ist die Ausnehmung zu einer zumindest teilweisen Aufnahme der UV-Lichtquelle vorgesehen. Vorzugsweise ist die Ausnehmung innerhalb der Führungsfläche des Ventilgehäuses angeordnet. Insbesondere sind die Ausnehmung begrenzende Seitenflächen und/oder die Innenfläche des Ventilgehäuses von der Führungsfläche begrenzt. Insbesondere grenzt die Ausnehmung an den Fluidkanal.

Zudem wird vorgeschlagen, dass die Sterilisierungseinheit ein Fensterelement umfasst, das für UV-Strahlung der zumindest einen UV-Lichtquelle durchlässig ausgebildet ist, wobei das Fensterelement dazu vorgesehen ist, die zumindest eine UV-Lichtquelle, insbesondere fluiddicht und/oder druckdicht, von dem Fluidkanal zu trennen. Es kann ein direkter Kontakt der UV-Lichtquelle mit dem Prozessmedium vorteilhaft verhindert werden. Dadurch können ungewollte Ablagerungen und/oder Beschädigungen der UV-Lichtquelle über das Prozessmedium vorteilhaft verhindert werden. Zudem können vorteilhafte Strömungseigenschaften des Ventilgehäuses ermöglicht werden, insbesondere da das Fensterelement bündig mit einer Innenwand des Ventilgehäuses ausgebildet werden kann. Bevorzugt ist das Fensterelement für die von der UV-Lichtquelle zur Sterilisierung erzeugte Strahlung, insbesondere mit einer Wellenlänge zwischen 100 nm und 280 nm, vorzugsweise zwischen 200 nm und 270 nm und besonders bevorzugt zwischen 250 nm und 260 nm, zumindest zu einem Großteil durchlässig ausgebildet. Bevorzugt ist das Fensterelement für die von der UV-Lichtquelle zur Sterilisierung erzeugte Strahlung im Wesentlichen vollständig durchlässig ausgebildet, wobei insbesondere mindestens mehr als 90%, vorzugsweise mehr als 95% und besonders bevorzugt mehr als 97%, der Strahlung das Fensterelement durchdringen kann. Es ist denkbar, dass das Fensterelement an dem Ventilgehäuse und/oder dem Trägerelement angeordnet ist. Vorzugsweise verdeckt das Fensterelement die in der Ausnehmung angeordnete UV-Lichtquelle gegenüber dem Fluidkanal im Wesentlichen vollständig, insbesondere fluiddicht und/oder druckdicht. Bevorzugt verschließt das Fensterelement die Ausnehmung gegenüber dem Fluidkanal fluiddicht und/oder druckdicht. Bevorzugt ist das Fensterelement zumindest größtenteils, insbesondere im Wesentlichen vollständig, innerhalb der Ausnehmung angeordnet. Vorzugsweise ist das Fensterelement derart ausgebildet und/oder angeordnet, dass eine dem Fluidkanal zugewandte Außenfläche des Fensterelements im Wesentlichen bündig mit der Führungsfläche des Ventilgehäuses ausgebildet ist. Alternativ oder zusätzlich ist denkbar, dass das Fensterelement die an dem Trägerelement angeordnete UV-Lichtquelle im Wesentlichen vollständig, insbesondere fluiddicht und/oder druckdicht, umschließt. Vorzugsweise ist das Fensterelement dazu vorgesehen, in zumindest einem, insbesondere Prozessmedium führenden, Betriebszustand des Stellventils einen direkten Kontakt der zumindest einen UV-Lichtquelle mit dem innerhalb des Fluidkanals geführten Prozessmediums zu verhindern. Beispielsweise ist das Fensterelement aus einem zumindest teilweise transparenten Kunststoff ausgebildet. Es sind aber auch andere Ausgestaltungen des Fensterelements denkbar, insbesondere aus einem für die UV-Strahlung im Wesentlichen durchlässigen Material.

Es ist denkbar, dass das Stellventil, insbesondere das Ventilgehäuse, eine Beschichtung und/oder eine Auskleidung aufweist, welche innerhalb eines Grundkörpers des Ventilgehäuses angeordnet sind/ist. Beispielsweise gibt es Membranventile mit einer Kunststoff-Beschichtung in einem Innenraum. Vorzugsweise bildet die Beschichtung bzw. die Auskleidung die Innenfläche(n) des Ventilgehäuses aus. Es ist denkbar, dass die Beschichtung und/oder die Auskleidung das Fensterelement ausbildet/ausbilden, insbesondere über einen zumindest für UV-Strahlung im Wesentlichen transparent ausgebildeten Bereich, oder, dass das Fensterelement in die Beschichtung und/oder die Auskleidung eingelassen ist. Besonders ist denkbar, dass die Beschichtung und/oder die Auskleidung als Befestigungsmittel für die UV-Lichtquelle und/oder das Fensterelement vorgesehen sind/ist, wobei insbesondere die UV-Lichtquelle und/oder das Fensterelement formschlüssig von der Beschichtung und/oder der Auskleidung gehalten werden/wird.

Ferner wird vorgeschlagen, dass die zumindest eine UV-Lichtquelle in einem Nahbereich der Drosselstelle angeordnet ist und/oder derart angeordnet ist, dass eine, insbesondere die vorher genannte, Hauptabstrahlrichtung der UV-Lichtquelle auf die Drosselstelle und/oder auf den Nahbereich der Drosselstelle gerichtet ist. Es kann eine vorteilhaft effiziente Bestrahlung von das Stellventil durchströmendem Prozessmedium ermöglicht werden, insbesondere da ein Querschnitt des Fluidkanals bzw. des Stellventils an der Drosselstelle vorteilhaft gering ausgebildet ist. Das Stellventil umfasst vorzugsweise einen Ventilsitz, der den Fluidkanal im Bereich der Drosselstelle zumindest teilweise begrenzt. Der Ventilsitz ist für ein Verschließen der Drosselstelle zu einem Zusammenwirken mit dem Ventilglied vorgesehen, wobei insbesondere das Ventilglied zu einer Anordnung an bzw. innerhalb des Ventilsitzes vorgesehen ist. Es ist denkbar, dass der Ventilsitz einstückig mit dem Ventilgehäuse ausgebildet bzw. von dem Ventilgehäuse ausgebildet wird oder, insbesondere abnehmbar, an dem Ventilgehäuse angeordnet, insbesondere befestigt, ist. Die Drosselstelle ist bevorzugt zwischen dem Ventilsitz und dem Ventilglied ausgebildet. Unter einem "Nahbereich" eines Objekts, insbesondere der Drosselstelle, soll vorzugsweise, insbesondere hinsichtlich einer Anordnung einer UV-Lichtquelle, ein Bereich verstanden werden, der sich in einem minimalen Abstand von höchstens 10 cm, vorzugsweise höchstens 8 cm und besonders bevorzugt höchstens 5 cm, um das Objekt, insbesondere die Drosselstelle oder eine die Drosselstelle bildende Dichtkante des Ventilsitzes, erstreckt. Vorzugsweise ist die im Nahbereich des Objekts angeordnete UV-Lichtquelle derart ausgebildet und/oder angeordnet, dass die von der UV-Lichtquelle erzeugte UV-Strahlung, insbesondere unabhängig von einem Öffnungsgrad des Stellventils, zu einem Sterilisieren des Objekts bzw. einem Bereich zwischen dem Objekt und der UV-Lichtquelle, insbesondere von durch die Drosselstelle strömendem Prozessmedium, geeignet ist, insbesondere hinsichtlich einer Strahlungsintensität und dem zu verwendenden Prozessmedium.

Bevorzugt ist die zumindest eine UV-Lichtquelle derart ausgebildet und/oder angeordnet, dass die Drosselstelle eine gesamte Querschnittsfläche der Drosselstelle im Wesentlichen gleichmäßig bestrahlt. Darunter, dass die "Hauptabstrahlrichtung der UV-Lichtquelle auf die Drosselstelle und/oder auf den Nahbereich der Drosselstelle gerichtet ist" soll insbesondere verstanden werden, dass die UV-Lichtquelle derart angeordnet ist, dass die Drosselstelle von der UV-Lichtquelle aus innerhalb eines kegelförmigen Bereichs angeordnet ist, der sich mit einem Winkel von höchstens 90°, vorzugsweise höchstens 60° und besonders bevorzugt höchstens 45°, um die Hauptabstrahlrichtung der UV-Lichtquelle erstreckt. In einer bevorzugten Ausgestaltung ist die UV-Lichtquelle derart angeordnet, dass die Hauptabstrahlrichtung der UV-Lichtquelle zumindest bereichsweise zwischen dem Ventilglied und dem Ventilsitz hindurch verläuft. Bevorzugt ist die UV-Lichtquelle derart angeordnet, dass die Hauptabstrahlrichtung der UV-Lichtquelle eine Mittelachse der Drosselstelle schneidet. In einer Ausgestaltung der Sterilisierungseinheit mit mehr als einer im Nahbereich der Drosselstelle angeordneten UV-Lichtquelle sind die UV-Lichtquellen vorzugsweise im Wesentlichen gleichmäßig um die Mittelachse der Drosselstelle verteilt angeordnet.

Des Weiteren wird vorgeschlagen, dass die Sterilisierungseinheit eine, insbesondere die vorher genannte, Versorgungseinheit zu einer elektrischen Versorgung der zumindest einen UV-Lichtquelle umfasst, wobei das Ventilgehäuse eine Durchführung ausbildet, die zu einer Verbindung der Versorgungseinheit mit der UV-Lichtquelle vorgesehen ist. Es kann eine vorteilhaft direkte Versorgung der Sterilisierungseinheit in dem Ventilgehäuse ermöglicht werden. Die Versorgungseinheit umfasst vorzugsweise einen Energiespeicher oder eine Verbindung zu einem Energiespeicher, wie beispielsweise ein Stromnetz o.dgl. Bevorzugt umfasst die Versorgungseinheit zumindest ein Übertragungselement zu einer Übertragung elektrischer Energie von dem Energiespeicher an die zumindest eine UV-Lichtquelle. Es ist denkbar, dass sich das Übertragungselement von außerhalb des Ventilgehäuses durch die Durchführung hindurch bis zu der zumindest einen UV-Lichtquelle hin erstreckt. Beispielsweise ist das Übertragungselement als ein Kabel, ein Draht o.dgl. ausgebildet. Bevorzugt erstreckt sich die Durchführung von einer Außenseite des Ventilgehäuses durch eine Wandung des Ventilgehäuses bis zu dem Fluidkanal und/oder der zumindest einen UV-Lichtquelle. Bevorzugt umfasst das Stellventil zumindest ein Dichtungsmittel, beispielsweise eine Dichtungspackung, ein Dichtring o.dgl., das dazu vorgesehen ist, die Durchführung mit dem Übertragungselement für das Prozessmedium aus dem Fluidkanal und/oder Gasen von außen abzudichten. Bevorzugt ist das Dichtungsmittel zumindest teilweise innerhalb oder an der Durchführung angeordnet. Vorzugsweise verdeckt das Dichtungsmittel die Durchführung zusammen mit dem Übertragungselement vollständig, insbesondere fluiddicht und druckdicht. Bevorzugt ist die Durchführung in das im Wesentlichen einteilig ausgebildete Ventilgehäuse gefräst, gebohrt oder durch ein anderes spanendes Fertigungsverfahren in das Ventilgehäuse eingebracht. In einer Ausgestaltung der Sterilisierungseinheit mit mehr als einer UV-Lichtquelle ist denkbar, dass die Versorgungseinheit mehrere Übertragungselemente umfasst, die jeweils mit einer der UV-Lichtquellen verbunden sind. Vorzugsweise ist die Durchführung dazu vorgesehen, alle Übertragungselemente der Versorgungseinheit in den Fluidkanal und/oder jeweils zu den UV-Lichtquellen zu führen. Alternativ ist denkbar, dass das Ventilgehäuse eine Mehrzahl von Durchführungen ausbildet, die jeweils zu einer Führung von einem oder mehreren Übertragungselementen vorgesehen sind.

Zudem wird vorgeschlagen, dass die Sterilisierungseinheit eine Versorgungseinheit zu einer elektrischen Versorgung der zumindest einen UV-Lichtquelle umfasst, wobei die Versorgungseinheit dazu vorgesehen ist, elektrische Energie zu einer Versorgung der UV-Lichtquelle aus dem Prozessmedium, insbesondere einer kinetischen und/oder thermischen Energie des Prozessmediums, umzuwandeln. Es kann eine zusätzliche externe Versorgungsleitung vorteilhaft entfallen. Es kann eine vorteilhaft autarke Sterilisierungseinheit ermöglicht werden. Es können zusätzliche Durchführungen in dem Ventilgehäuse vorteilhaft entfallen. Dadurch können vorteilhaft geringe Herstellungs- und Montagekosten erreicht werden. Bevorzugt umfasst die Versorgungseinheit zumindest einen Umwandler, der dazu vorgesehen ist, kinetische oder thermische Energie des Prozessmediums in elektrische Energie umzuwandeln. Insbesondere ist der Umwandler direkt oder mittels einem Übertragungselement der Versorgungseinheit mit der zumindest einen UV-Lichtquelle verbunden. Beispielsweise weist der Umwandler zur Umwandlung von kinetischer Energie einen Generator und ein als Rotor, Rad oder dergleichen ausgebildetes Anregungselement auf. Vorzugsweise ist der Umwandler zur Umwandlung von kinetischer Energie, insbesondere zumindest das Anregungselement, zumindest teilweise innerhalb des Ventilgehäuses bzw. des Fluidkanals angeordnet. Beispielsweise weist der Umwandler zur Umwandlung von thermischer Energie zumindest ein Thermoelement auf. Insbesondere ist der Umwandler zur Umwandlung von thermischer Energie, insbesondere zumindest das Thermoelement, zumindest teilweise innerhalb des Ventilgehäuses bzw. des Fluidkanals angeordnet. Alternativ ist denkbar, dass der Umwandler als ein Wärmetauscher ausgebildet ist, wobei insbesondere ein weiterer Umwandler der Versorgungseinheit dazu vorgesehen ist, eine Bewegung eines über der Umwandler erhitzten Mediums, beispielsweise Dampf o.dgl., in elektrische Energie umzuwandeln, wobei insbesondere der weitere Umwandler mit der zumindest einen UV-Lichtquelle verbunden ist. Beispielsweise weist der weitere Umwandler ein Generator und ein als Rotor, Rad oder dergleichen ausgebildetes Anregungselement auf. Vorzugsweise ist denkbar, dass die Versorgungseinheit, insbesondere der/die Umwandler, zumindest größtenteils, vorzugsweise im Wesentlichen vollständig, innerhalb des Ventilgehäuses, insbesondere des Fluidkanals, angeordnet ist/sind.

Ferner wird vorgeschlagen, dass das Stellventil zumindest eine Pneumatik-Leitung zu einem Betrieb der Antriebseinheit und/oder eines Stellungsreglers des Stellventils aufweist, wobei die Sterilisierungseinheit eine Versorgungseinheit zu einer elektrischen Versorgung der zumindest einen UV-Lichtquelle umfasst, wobei die Versorgungseinheit dazu vorgesehen ist, aus einer Strömung in der Pneumatik-Leitung Energie zu einem Betrieb der UV-Lichtquelle umzuwandeln. Es kann eine zusätzliche externe Versorgungsleitung vorteilhaft entfallen. Es kann vorteilhaft ein Betrieb der Sterilisierungseinheit ohne zusätzliche Hilfsenergie ermöglicht werden, insbesondere da das Stellventil bereits eine Pneumatik-Zuluft benötigt. Vorzugsweise umfasst die Versorgungseinheit zumindest einen Pneumatik-Umwandler, welcher an der Pneumatik-Leitung angeordnet ist. Der Pneumatik-Umwandler ist bevorzugt zu einer Umwandlung von pneumatischer Energie in elektrische Energie vorgesehen. Insbesondere umfasst der Pneumatik-Umwandler zumindest ein Anregungselement, welches dazu vorgesehen ist, durch die Strömung in der Pneumatik-Leitung bewegt zu werden. Bevorzugt umfasst der Pneumatik-Umwandler zumindest einen Generator o.dgl. zu einer Umwandlung der Bewegung des Anregungselements in elektrische Energie. Alternativ ist denkbar, dass der Pneumatik-Wandler dazu vorgesehen ist, insbesondere unabhängig von einer kontinuierlich bewirkten Bewegung, einen Druck innerhalb der Pneumatik-Leitung in elektrische Energie umzuwandeln. Beispielsweise umfasst der Pneumatik-Wandler zumindest ein Piezoelektrisches Element, welches in oder an der Pneumatik-Leitung angeordnet ist. Vorzugsweise ist die Pneumatik-Leitung und/oder der Pneumatik-Umwandler außerhalb des Fluidkanals bzw. des Ventilgehäuses angeordnet. Beispielsweise ist die Pneumatik-Leitung als Zuluft- oder AbluftLeitung der Antriebseinheit oder des Stellungsreglers des Stellventils ausgebildet. Bevorzugt ist der Pneumatik-Umwandler über ein Übertragungselement der Versorgungseinheit elektrisch mit der zumindest einen UV-Lichtquelle verbunden, insbesondere über die/eine Durchführung.

Des Weiteren wird vorgeschlagen, dass die Sterilisierungseinheit eine Versorgungseinheit zu einer elektrischen Versorgung der zumindest einen UV-Lichtquelle umfasst, wobei die Versorgungseinheit dazu vorgesehen ist, die zumindest eine UV-Lichtquelle kabellos, insbesondere durch zumindest eine Wandung des Ventilgehäuses, mit elektrischer Energie zu versorgen. Es können zusätzliche Durchführungen in dem Ventilgehäuse vorteilhaft entfallen. Dadurch können vorteilhaft geringe Herstellungs- und Montagekosten erreicht werden. Es kann eine vorteilhaft hohe Flexibilität bei einer Anordnung der UV-Lichtquelle an dem Ventilgehäuse erreicht werden, insbesondere da diese unabhängig von einer Durchführung und/oder Verkabelung durch das Ventilgehäuse hindurch versorgt werden kann. Insbesondere kann die kabellose Verbindung zwischen der Versorgungseinheit und der zumindest einen UV-Lichtquelle auch als kontaktlose Verbindung bezeichnet werden. Die kabellose Verbindung der Versorgungseinheit und der zumindest einen UV-Lichtquelle ist vorzugsweise durch eine induktive Kopplung realisiert. Es sind jedoch auch andere kabellose Verbindungsarten zwischen der Versorgungseinheit und der zumindest einen UV-Lichtquelle denkbar, wie über eine kapazitive Kopplung oder über elektromagnetische Wellen, insbesondere über Licht oder Funksignale. Bevorzugt umfasst die Versorgungseinheit ein an der zumindest einen UV-Lichtquelle, insbesondere der UV-Lichtquelle, angeordnetes bzw. mit der UV-Lichtquelle direkt verbundenes Verbindungselement und ein weiteres Verbindungselement, welches getrennt von der UV-Lichtquelle und von dem Verbindungselement angeordnet ist. Vorzugsweise sind das Verbindungselement und das weitere Verbindungselement dazu vorgesehen, untereinander Energie zu übertragen, bevorzugt zumindest von dem weiteren Verbindungselement zu dem Verbindungselement. Bevorzugt ist das Verbindungselement an einer/der Innenseite bzw. der Innenwand des Ventilgehäuses angeordnet, insbesondere in die Innenwand integriert. Insbesondere ist das Verbindungselement innerhalb der Ausnehmung angeordnet. Insbesondere ist denkbar, dass das Verbindungselement innerhalb des Fluidkanals angeordnet ist. Das weitere Verbindungselement ist vorzugsweise an der/einer Außenseite des Ventilgehäuses angeordnet. Bevorzugt ist zumindest eine Wandung des Ventilgehäuses zwischen dem Verbindungselement und dem weiteren Verbindungselement angeordnet. Es ist denkbar, dass die Versorgungseinheit eine Mehrzahl von Verbindungselementen und/oder weiteren Verbindungselementen umfasst, insbesondere zu einer Versorgung von mehreren UV-Lichtquellen. Alternativ ist denkbar, dass mehrere UV-Lichtquellen zu einer elektrischen Versorgung mit einem Verbindungselement der Versorgungseinheit verbunden sind. Beispielsweise sind das Verbindungselement und das weitere Verbindungselement jeweils als eine Spule ausgebildet. Es sind jedoch, insbesondere je nach Art der Verbindung, auch andere Ausgestaltungen der Verbindungselemente denkbar, beispielsweise als Emitter und Empfänger oder als Kondensatorplatten. Bevorzugt umfasst die Versorgungseinheit Abschirmmittel zu einer elektrischen Abschirmung der Verbindungselemente, insbesondere des Verbindungselements und/oder des weiteren Verbindungselements, zu dem Ventilgehäuse. Beispielsweise sind die Abschirmmittel als ein Verguss, als eine Hülse, insbesondere eine Kunststoff- oder Gummihülse, o.dgl. ausgebildet. Insbesondere sind die Abschirmmittel dazu vorgesehen, einen elektrischen Kontakt zwischen den/dem jeweiligen Verbindungsmittel(n) und dem Ventilgehäuse zu verhindern.

Zudem wird vorgeschlagen, dass das Ventilgehäuse zumindest einen, insbesondere den vorher genannten, Fluidkanal für das Prozessmedium ausbildet, der zumindest teilweise von der Drosselstelle begrenzt ist, wobei die zumindest eine UV-Lichtquelle in einem Bereich des Fluidkanals mit einer zu erwartenden minimalen lokalen Strömungsgeschwindigkeit des Prozessmediums angeordnet ist und/oder derart angeordnet ist, dass eine Hauptabstrahlrichtung der UV-Lichtquelle auf den Bereich des Fluidkanals mit einer zu erwartenden minimalen lokalen Strömungsgeschwindigkeit des Prozessmediums gerichtet ist. Es kann vorteilhaft eine ungewollte Keimbildung innerhalb des Ventilgehäuses verhindert werden. Es kann eine vorteilhaft hohe Flexibilität des Stellventils hinsichtlich einer Viskosität des zu verwendenden Prozessmediums ermöglicht werden. Es ist denkbar, dass das Ventilgehäuse, insbesondere der vom Ventilgehäuse gebildete Fluidkanal, zu einer Minimierung von Kavitation, zu einer Optimierung des Durchflusseigenschaften o.dgl. speziell ausgebildet wird, wobei an manchen Stellen im Fluidkanal, beispielsweise in einer Kammer vor der Drosselstelle oder nach der Drosselstelle, lokale Minima für die Strömungsgeschwindigkeit des Prozessmediums auftreten können. Durch die lokal reduzierte Strömungsgeschwindigkeit des Prozessmediums können im Prozessmedium an diesen Stellen andere Eigenschaften des Prozessmediums, beispielsweise abweichende Temperaturbedingungen, beobachtet werden und gegebenenfalls leichter Keime entstehen. Durch die Anordnung bzw. Ausrichtung der UV-Lichtquelle an/auf diese Stellen kann eine Keimbildung vorteilhaft verhindert werden.

Des Weiteren wird vorgeschlagen, dass das Ventilgehäuse zumindest einen, insbesondere den vorher genannten, Fluidkanal für das Prozessmedium ausbildet, der zumindest teilweise von der Drosselstelle begrenzt ist, wobei das Ventilgehäuse an einer den Fluidkanal begrenzenden Innenfläche eine Vertiefung und/oder eine Schweißnaht aufweist, wobei die zumindest eine UV-Lichtquelle in einem Nahbereich der Vertiefung und/oder der Schweißnaht angeordnet ist und/oder derart angeordnet ist, dass eine Hauptabstrahlrichtung der UV-Lichtquelle auf die Vertiefung und/oder die Schweißnaht gerichtet ist. Es können Stellen innerhalb des Fluidkanals, an denen sich Prozessmedium staut oder üblicherweise absetzt vorteilhaft sterilisiert gehalten werden. Es kann vorteilhaft eine ungewollte Keimbildung innerhalb des Ventilgehäuses verhindert werden. Vorzugsweise ist die Vertiefung und/oder die Schweißnaht an der Führungsfläche angeordnet oder bilden die Führungsfläche teilweise aus. Es ist denkbar, dass die UV-Lichtquelle an einer der Vertiefung und/oder eine Schweißnaht gegenüberliegenden Innenfläche des Stellventils, insbesondere des Ventilgehäuses, angeordnet ist. Alternativ oder zusätzlich ist denkbar, dass die UV-Lichtquelle zumindest teilweise innerhalb des Fluidkanals angeordnet ist und mit der Hauptabstrahlrichtung in einem von 90° verschiedenen Winkel zur jeweiligen Innenfläche, auf der die UV-Lichtquelle angeordnet ist, ausgerichtet ist.

Ferner wird vorgeschlagen, dass das Ventilgehäuse eine Verbindungsstelle, insbesondere einen Flansch und/oder eine Dichtungskante, umfasst, wobei die zumindest eine UV-Lichtquelle in einem Nahbereich der Verbindungsstelle angeordnet ist und/oder mit einer Hauptabstrahlrichtung auf die Verbindungsstelle und/oder auf den Nahbereich der Verbindungsstelle gerichtet ist. Es kann eine ungewollte Entstehung von Keimen an Kanten und Nuten, an denen sich für gewöhnlich Prozessmedium und/oder Rückstände absetzen, vorteilhaft verhindert werden. Es kann eine vorteilhaft hohe Reinlichkeit innerhalb des Stellventils erreicht werden. Bevorzugt weist das Ventilgehäuse im Bereich der Eintrittsöffnung und im Bereich der Austrittsöffnung jeweils eine Verbindungsstelle, insbesondere einen Flansch, wie einen Rohrflansch o.dgl., auf. Vorzugsweise bildet die Dichtkante eine Kontaktstelle zwischen zwei Bauteilen des Stellventils oder zwischen dem Stellventil und einem anderen Bauteil der Prozessanlage aus. Es ist denkbar, dass das Ventilgehäuse beabstandet von der Eintrittsöffnung und der Austrittsöffnung eine Verbindungsstelle und/oder eine Dichtkante ausbildet, beispielsweise zu einer Verbindung eines Ventilaufbaus, insbesondere des Antriebs, des Stellungsreglers o.dgl., des Stellventils mit dem Ventilgehäuse. Alternativ oder zusätzlich ist denkbar, dass die UV-Lichtquelle, die Ausnehmung und/oder die Durchführung zur Versorgung der UV-Lichtquelle in eine Verbindungsstelle, insbesondere einen Flansch, des Ventilgehäuses integriert ist. Beispielsweise erstreckt sich die Durchführung im Bereich der Verbindungsstelle, insbesondere radial zu einer Mittelachse der Verbindungsstelle, von außen bis zu dem Fluidkanal.

Des Weiteren wird vorgeschlagen, dass die Sterilisierungseinheit eine Versorgungseinheit zu einer elektrischen Versorgung der zumindest einen UV-Lichtquelle umfasst und das Ventilgehäuse eine Versorgungs-Verbindungsstelle, insbesondere einen Flansch, umfasst, wobei die Versorgungseinheit dazu vorgesehen ist, die UV-Lichtquelle durch die Versorgungs-Verbindungsstelle hindurch mit elektrischer Energie zu versorgen. Es kann eine vorteilhaft einfache und direkte Befestigung der Versorgungseinheit am Ventilgehäuse ermöglicht werden. Es kann eine vorteilhaft hohe Modularität und eine einfache Wartung des Stellventils erreicht werden, insbesondere da die Sterilisierungseinheit und/oder die Versorgungseinheit über die Verbindungsstelle vorteilhaft zugänglich ausgebildet und vorteilhaft einfach ausgetauscht werden können. Bevorzugt umfasst das Stellventil, insbesondere die Versorgungseinheit, einen Versorgungsverbinder, welcher zu einer Befestigung an der Versorgungs-Verbindungsstelle. Insbesondere ist der Versorgungsverbinder korrespondierend zu der Versorgungs-Verbindungsstelle ausgebildet. Der Versorgungsverbinder ist vorzugsweise dazu vorgesehen, ein Übertragungselement der Versorgungseinheit von außen in den Fluidkanal zu führen. Bevorzugt ist der Versorgungsverbinder dazu vorgesehen, das Übertragungselement gegenüber dem Ventilgehäuse elektrisch zu isolieren. Besonders bevorzugt ist der Versorgungsverbinder dazu vorgesehen, die Versorgungs-Verbindungsstelle im Wesentlichen vollständig zu verdecken und insbesondere für das Prozessmedium fluiddicht und/oder druckdicht zu verschließen. Es ist denkbar, dass das Stellventil ein Abdeckteil umfasst, dass dazu vorgesehen ist, die Versorgungs-Verbindungsstelle in Abwesenheit einer UV-Lichtquelle bzw. des Versorgungsverbinders abzudecken, insbesondere fluiddicht und/oder druckdicht zu verschließen. Dadurch kann eine vorteilhaft hohe Modularität und Funktionalität des Tellventils erreicht werden, da vorteilhaft einfach verschiedene Aufbauten - mit und ohne Versorgung bzw. UV-Lichtquelle - umgesetzt werden können. Insbesondere kann gegenüber einer Ausgestaltung des Ventilgehäuses mit Durchführung vorteilhaft einfacher eine modulare Anordnung der Sterilisierungseinheit, insbesondere von UV-Lichtquellen, ermöglicht werden.

Alternativ oder zusätzlich ist denkbar, dass die Versorgungseinheit ein Übertragungselement umfasst, welches durch einen Ventilaufbau des Stellventils, welcher vorzugsweise an dem Ventilgehäuse befestigt bzw. befestigbar ist, von außen in den Fluidkanal geführt ist. Insbesondere umfasst der Ventilaufbau eine Verbindungsschnittstelle zu einer Befestigung am Ventilgehäuse. Insbesondere ist die Ventilstange in der Verbindungsschnittstelle beweglich gelagert und/oder geführt. Bevorzugt ist die Antriebseinheit als Teil des Ventilaufbaus ausgebildet und, vorzugsweise über einen Rahmen o.dgl., an der Verbindungsschnittstelle angeordnet. Das Ventilgehäuse bildet vorzugsweise eine Verbindungsstelle zur Verbindung mit dem Ventilaufbau, insbesondere der Verbindungsschnittstelle, aus. Die Verbindungsschnittstelle ist vorzugsweise als ein Flansch ausgebildet und insbesondere dazu vorgesehen, vorzugsweise fluiddicht und/oder druckdicht, mittels Befestigungsmitteln, wie Schrauben, Klemmen o.dgl., an dem Ventilgehäuse befestigt zu werden. Bevorzugt umfasst die Verbindungsschnittstelle eine Durchführung für das/ein Übertragungselement der Versorgungseinheit. Vorzugsweise ist das Übertragungselement innerhalb des Fluidkanals an die UV-Lichtquelle geführt und mit der UV-Lichtquelle verbunden. Bevorzugt umfasst die Verbindungsschnittstelle eine Isolierung zu einer elektrischen Isolierung der Durchführung bzw. des Übertragungselements von einem Grundkörper der Verbindungsschnittstelle und/oder anderen Bauteilen der Verbindungsschnittstelle.

Das erfindungsgemäße Stellventil soll hierbei nicht auf die oben beschriebene Anwendung und Ausführungsform beschränkt sein. Insbesondere kann das erfindungsgemäße Stellventil zu einer Erfüllung einer hierin beschriebenen Funktionsweise eine von einer hierin genannten Anzahl von einzelnen Elementen, Bauteilen und Einheiten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

### Zeichnungen

Weitere Vorteile ergeben sich aus der folgenden Zeichnungsbeschreibung. In den Zeichnungen sind drei6 Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Figur 1.:: eine schematische Schnittansicht eines erfindungsgemäßen als Membranventil ausgebildeten Stellventils für eine Prozessanlage mit einer Sterilisierungseinheit,
- Figur 2.:: eine schematische Schnittansicht einer alternativen Ausgestaltung eines erfindungsgemäßen Stellventils mit verschiedenen Anordnungen einer UV-Lichtquelle einer Sterilisierungseinheit des Stellventils sowie einer Versorgungseinheit zur Umwandlung von pneumatischer Energie und
- Figur 3.:: eine schematische Schnittansicht einer weiteren alternativen Ausgestaltung eines erfindungsgemäßen Stellventils mit einer modular bestückbaren Verbindungsstelle am Ventilgehäuse.

### Beschreibung der Ausführungsbeispiele

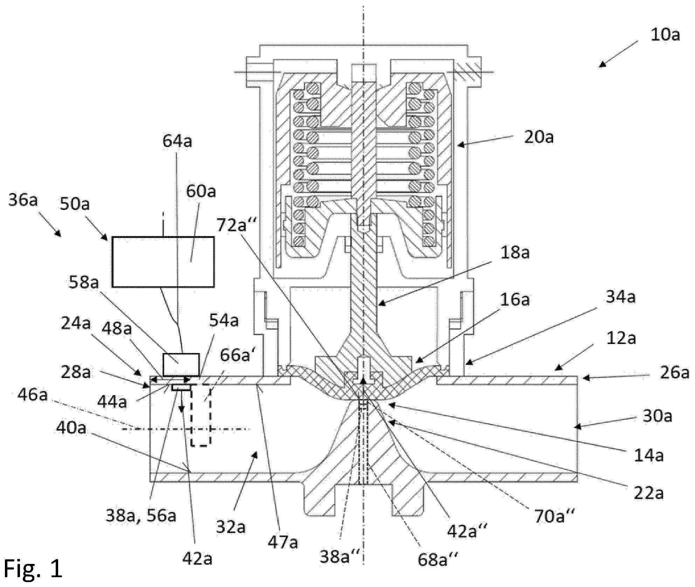

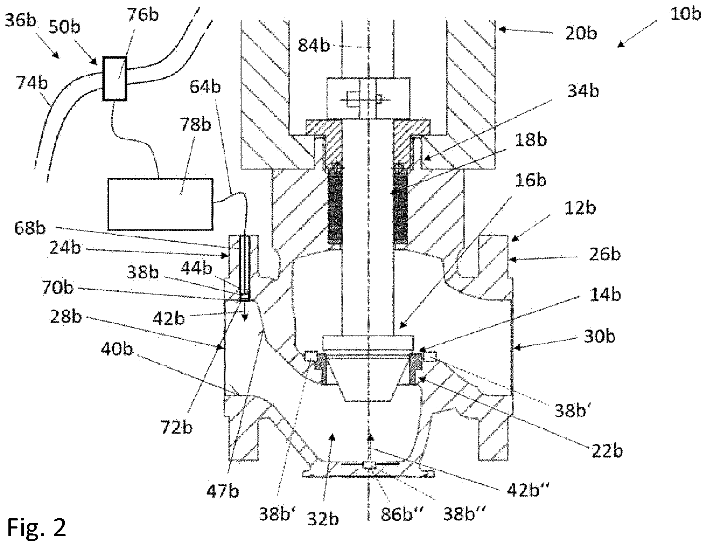

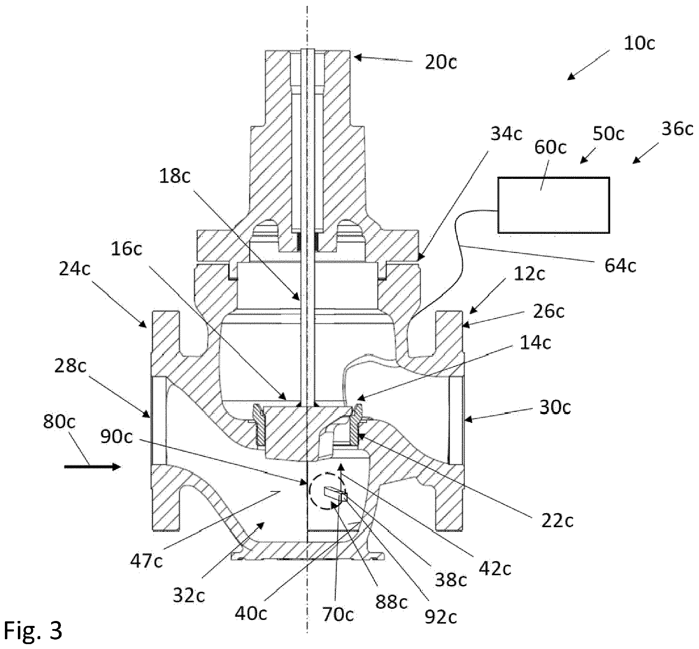

In Figur 1 ist ein Stellventil 10a für eine Prozessanlage gezeigt. Das Stellventil 10a ist als ein Membranventil ausgebildet. Es sind aber auch andere Ausgestaltungen des Stellventils 10a denkbar (vgl. bspw. Figuren 2 und 3). Das Stellventil 10a ist für eine Prozessanlage mit hohen Reinlichkeitsanforderungen, beispielsweise in der Lebensmittel- und/oder der Pharmaindustrie, vorgesehen. Das Stellventil 10a umfasst ein Ventilgehäuse 12a, das eine Drosselstelle 14a für ein Prozessmedium ausbildet. Insbesondere ist die Drosselstelle 14a in Figur 1 in einem verschlossenen Zustand gezeigt. Das Stellventil 10a umfasst ein Ventilglied 16a, das als eine flexible Membran ausgebildet ist. Das Stellventil 10a umfasst eine Ventilstange 18a und eine Antriebseinheit 20a, die dazu vorgesehen ist, das Ventilglied 16a über die Ventilstange 18a in einem Bereich der Drosselstelle 14a zu bewegen. Das Ventilgehäuse 12a weist einen Ventilsitz 22a auf, welcher als eine Erhebung ausgebildet ist und sich im Bereich des Ventilglieds 16a entlang einer Bewegungsachse des Ventilglieds 16a, der Ventilstange 18a bzw. der Antriebseinheit 20a erstreckt. Die Drosselstelle 14a ist zwischen dem Ventilsitz 22a und dem Ventilglied 16a, insbesondere der Membran, ausgebildet. Das als Membran ausgebildete Ventilglied 16a ist dazu vorgesehen, zu einem Verschließen bzw. einem Reduzieren eines Querschnitts der Drosselstelle 14a auf den Ventilsitz 22a bewegt zu werden, wobei sich insbesondere das als Membran ausgebildete Ventilglied 16a verformt. Die Ventilstange 18a weist eine Haupterstreckungsachse auf, wobei die Ventilstange 18a zu einem Schließen oder Öffnen des Stellventils 10a entlang der Haupterstreckungsachse bewegbar ist. Alternativ sind auch Ausgestaltungen des Stellventils 10a als Drehventil, wie beispielsweise ein Kugelhahnventil oder ein Klappenventil, denkbar, wobei insbesondere die Ventilstange zu einem Schließen des Stellventils um eine Achse gedreht wird. Insbesondere wird bei einer Bewegung der Ventilstange 18a entlang der Haupterstreckungsachse das Ventilglied 16a auf den Ventilsitz 22a bewegt.

Das Ventilgehäuse 12a und der Ventilsitz 22a sind einstückig ausgebildet. Das Ventilgehäuse 12a umfasst zwei Verbindungsstellen 24a, 26a jeweils an einer Eintrittsöffnung 28a und an einer Austrittsöffnung 30a des Ventilgehäuses 12a. Das Ventilgehäuse 12a begrenzt einen Fluidkanal 32a, welcher sich von der Eintrittsöffnung 28a durch die Drosselstelle 14a hindurch bis zu der Austrittsöffnung 30a erstreckt. Das Ventilgehäuse 12a umfasst eine weitere Verbindungsstelle 34a zu einer Befestigung eines Ventilaufbaus des Stellventils 10a, welcher insbesondere die Antriebseinheit 20a umfasst. Das als Membran ausgebildete Ventilglied 16a ist derart befestigt, dass das Ventilglied 16a den Fluidkanal 32a im Bereich der weiteren Verbindungsstelle 34a verschließt. Der Fluidkanal 32a ist im Bereich der Eintrittsöffnung 28a und im Bereich der Austrittsöffnung 30a im Wesentlichen gerade ausgebildet. Es sind aber auch andere Ausgestaltungen des Ventilgehäuses 12a denkbar. Das Ventilgehäuse 12a ist dazu vorgesehen, ein über die Eintrittsöffnung 28a einlaufendes Prozessmedium über die Drosselstelle 14a bis zur Austrittsöffnung 30a zu führen. Das Ventilgehäuse 12a erstreckt sich im Bereich der Drosselstelle 14a über einen Großteil einer Querschnittsfläche des Fluidkanals 32a an der Eintrittsöffnung 28a bzw. der Austrittsöffnung 30a. Der Ventilsitz 22a ist als eine Erhebung an einer dem Ventilglied 16a gegenüberliegenden Innenwand des Ventilgehäuses 12a ausgebildet, die sich insbesondere in Richtung des Ventilglieds 16a in den Fluidkanal 32a erstreckt.

Das Stellventil 10a umfasst eine Sterilisierungseinheit 36a zu einem Sterilisieren von Innenflächen des Stellventils 10a, insbesondere des Ventilgehäuses 12a, und/oder des Prozessmediums, wobei die Sterilisierungseinheit 36a eine UV-Lichtquelle 38a umfasst. Die UV-Lichtquelle 38a ist als eine LED ausgebildet und dazu vorgesehen, Strahlung im UVc-Bereich des elektromagnetischen Spektrums zu erzeugen. Die UV-Lichtquelle 38a ist dazu vorgesehen, das Prozessmedium bei einem Vorbeibewegen an der Eintrittsöffnung 28a zu sterilisieren. Zudem ist denkbar, dass die UV-Lichtquelle 38a dazu vorgesehen ist, den Fluidkanal 32a begrenzende Innenflächen 40a des Ventilgehäuses 12a zu sterilisieren. Vorzugsweise kann mittels der Sterilisierungseinheit 36a auch eine den Fluidkanal 32a begrenzende Innenfläche des als Membran ausgebildeten Ventilglieds 16a sterilisiert werden. Die Sterilisierungseinheit 36a ist zu einer im Wesentlichen kontaktfreien Sterilisierung der Innenflächen 40a und des Prozessmediums vorgesehen. Die UV-Lichtquelle 38a ist vollständig innerhalb des Ventilgehäuses 12a angeordnet. Die UV-Lichtquelle 38a ist mit Ausnahme der Eintrittsöffnung 28a und der Austrittsöffnung 30a im Wesentlichen vollständig von dem Ventilgehäuse 12a umschlossen. Die als UV-LED ausgebildete UV-Lichtquelle 38a weist eine maximale Längs- bzw. Quererstreckung von höchstens 0,5 cm auf. Alternativ sind auch andere Ausgestaltungen der UV-Lichtquelle 38a denkbar. Insbesondere sind Ausgestaltungen der Sterilisierungseinheit 36a mit mehr als einer UV-Lichtquelle 38a denkbar, die insbesondere an verschiedenen Stellen im Stellventil 10a, insbesondere im Ventilgehäuse 12a, angeordnet sind (vgl. bspw. Figuren 2 und 3). Die UV-Lichtquelle 38a ist derart angeordnet, dass eine Hauptabstrahlrichtung 42a der UV-Lichtquelle 38a von einer Innenfläche 44a des Ventilgehäuses 12a, an der die UV-Lichtquelle 38a angeordnet ist, in Richtung des Fluidkanals 32a ausgerichtet ist. Die UV-Lichtquelle 38a ist derart angeordnet, dass die Hauptabstrahlrichtung 42a der UV-Lichtquelle 38a von der Innenfläche 44a des Ventilgehäuses 12a, an der die UV-Lichtquelle 38a angeordnet ist, in Richtung einer der Innenfläche 44a gegenüberliegenden den Fluidkanal begrenzenden Innenwand des Ventilgehäuses 12a ausgerichtet ist. Bevorzugt ist die UV-Lichtquelle 38a derart angeordnet, dass die Hauptabstrahlrichtung 42a der UV-Lichtquelle 38a einen Bereich um eine Mittelachse 46a der Eintrittsöffnung 28a, die sich insbesondere zumindest teilweise innerhalb des Fluidkanals 32a erstreckt, schneidet.

Der Fluidkanal 32a ist teilweise von der Drosselstelle 14a begrenzt. Die UV-Lichtquelle 38a ist an der dem Fluidkanal 32a zugewandten und den Fluidkanal 32a begrenzenden Innenfläche 44a des Ventilgehäuses 12a angeordnet. Die UV-Lichtquelle 38a ist innerhalb des Fluidkanals 32a angeordnet. Die Innenfläche 44a des Ventilgehäuses 12a, an der die UV-Lichtquelle 38a angeordnet ist, ist Teil einer Führungsfläche 47a des Ventilgehäuses 12a, die insbesondere zumindest größtenteils den Fluidkanal 32a ausbildet. Die UV-Lichtquelle 38a ist innerhalb des Fluidkanals 32a angeordnet. Die Innenfläche 44a des Ventilgehäuses 12a, an der die UV-Lichtquelle 38a angeordnet ist, ist gebogen ausgebildet. Insbesondere weist der Fluidkanal 32a im Bereich der Eintrittsöffnung 28a eine im Wesentlichen kreisrunde Querschnittsfläche auf. Es sind aber auch andere Ausgestaltungen des Ventilgehäuses 12a, insbesondere des Fluidkanals 32a, denkbar, beispielsweise mit einer von einer kreisrunden Querschnittsfläche verschieden ausgebildeten Querschnittsfläche. Alternativ oder zusätzlich ist denkbar, dass die Innenfläche 44a des Ventilgehäuses 12a, an der die UV-Lichtquelle 38a angeordnet ist, im Wesentlichen eben ausgebildet ist und insbesondere von der Führungsfläche 47a abgesetzt ausgebildet ist. Dadurch kann eine bevorzugt einfachere Befestigung der UV-Lichtquelle 38a an dem Ventilgehäuse 12a, insbesondere der Innenfläche 44a, ermöglicht werden. Alternativ oder zusätzlich ist denkbar, dass das Ventilgehäuse 12a an einer den Fluidkanal 32a begrenzenden Innenwand eine Ausnehmung begrenzt, wobei die UV-Lichtquelle 38a zumindest teilweise, insbesondere im Wesentlichen vollständig, innerhalb der Ausnehmung angeordnet ist (vgl. Figur 2).

Die UV-Lichtquelle 38a ist in einem Nahbereich der im Bereich der Eintrittsöffnung 28a ausgebildeten Verbindungsstelle 24a des Ventilgehäuses 12a angeordnet. Alternativ oder zusätzlich ist denkbar, dass die UV-Lichtquelle 38a derart angeordnet ist, dass die Hauptabstrahlrichtung 42a der UV-Lichtquelle 38a auf die Verbindungsstelle 24a und/oder auf den Nahbereich der Verbindungsstelle 24a gerichtet ist. Die UV-Lichtquelle 38a ist innerhalb eines maximalen Abstands 48a von 3 cm von der Verbindungsstelle 24a angeordnet. Die UV-Lichtquelle 38a ist an einer dem Ventilaufbau, insbesondere der Antriebseinheit 20a, zugewandten Oberseite des Ventilgehäuses 12a angeordnet. Es sind jedoch auch andere Anordnungen der UV-Lichtquelle 38a denkbar, beispielsweise an einer gegenüber der Oberseite ausgebildeten Unterseite des Ventilgehäuses 12a, wobei insbesondere eine größere Menge an UV-Strahlung direkt auf das Ventilglied 16a gelangen kann. Es ist denkbar, dass die Sterilisierungseinheit 36a ein Fensterelement umfasst, das für UV-Strahlung der zumindest einen UV-Lichtquelle 38a durchlässig ausgebildet ist, wobei das Fensterelement dazu vorgesehen ist, die zumindest eine UV-Lichtquelle 38a, insbesondere fluiddicht und/oder druckdicht, von dem Fluidkanal 32a zu trennen.

Die Sterilisierungseinheit 36a umfasst eine Versorgungseinheit 50a zu einer elektrischen Versorgung der zumindest einen UV-Lichtquelle 38a. Die Versorgungseinheit 50a ist dazu vorgesehen, die UV-Lichtquelle 38a kabellos durch eine Wandung 54a des Ventilgehäuses 12a, die insbesondere die Innenfläche 44a ausbildet, mit elektrischer Energie zu versorgen. Die kabellose Verbindung der Versorgungseinheit 50a und der UV-Lichtquelle 38a ist durch eine induktive Kopplung realisiert. Es sind jedoch auch andere kabellose Verbindungsarten zwischen der Versorgungseinheit 50a und der zumindest einen UV-Lichtquelle 38a denkbar, wie über eine kapazitive Kopplung oder über elektromagnetische Wellen, insbesondere über Licht oder Funksignale. Die Versorgungseinheit 50a umfasst ein an der UV-Lichtquelle 38a angeordnetes bzw. mit der UV-Lichtquelle 38a direkt verbundenes Verbindungselement 56a und ein weiteres Verbindungselement 58a, welches getrennt von der UV-Lichtquelle 38a und von dem Verbindungselement 56a angeordnet ist. Das Verbindungselement 56a und das weitere Verbindungselement 58a sind jeweils als eine Spule ausgebildet. Es sind jedoch, insbesondere je nach Art der Verbindung, auch andere Ausgestaltungen der Verbindungselemente 56a, 58a denkbar, beispielsweise als Emitter und Empfänger oder als Kondensatorplatten. Das Verbindungselement 56a und das weitere Verbindungselement 58a sind dazu vorgesehen, untereinander Energie zu übertragen, bevorzugt zumindest von dem weiteren Verbindungselement 58a zu dem Verbindungselement 56a. Das Verbindungselement 56a ist an der UV-Lichtquelle 38a und einer Innenseite des Ventilgehäuses 12a angeordnet. Insbesondere sind die UV-Lichtquelle 38a und das Verbindungselement 56a als ein Bauteil ausgebildet. Alternativ ist denkbar, dass die UV-Lichtquelle 38a über das Verbindungselement 56a an der Innenfläche 44a oder getrennt von dem Verbindungselement 56a an dem Ventilgehäuse 12 angeordnet ist. Das Verbindungselement 56a ist zusammen mit der UV-Lichtquelle 38a innerhalb des Fluidkanals 32a angeordnet. Alternativ ist denkbar, dass das Verbindungselement 56a, insbesondere zusammen mit der UV-Lichtquelle 38a, in eine Wandung des Ventilgehäuses 12a integriert ist, beispielsweise innerhalb einer Ausnehmung. Das weitere Verbindungselement 58a ist an einer Außenseite des Ventilgehäuses 12a angeordnet. Die Wandung 54a des Ventilgehäuses 12a ist zwischen dem Verbindungselement 56a und dem weiteren Verbindungselement 58a angeordnet. Insbesondere umfasst die Versorgungseinheit 50a einen Energiespeicher 60a und/oder einen Anschluss an einen Energiespeicher, wie ein elektrisches Netz o.dgl. Bevorzugt ist die Versorgungseinheit 50a dazu vorgesehen, elektrische Energie aus dem Energiespeicher 60a über ein Übertragungselement 64a, wie ein Kabel, das weitere Verbindungselement 58a und das Verbindungselement 56a an die UV-Lichtquelle 38a zu übertragen. Alternativ ist denkbar, dass das Verbindungselement 56a innerhalb des Ventilgehäuses 12a über ein weiteres Übertragungselement mit einer oder mehreren UV-Lichtquellen 38a verbunden ist. Bevorzugt umfasst die Versorgungseinheit 50a Abschirmmittel zu einer elektrischen Abschirmung der Verbindungselemente 56a 58a, insbesondere des Verbindungselements 56a und des weiteren Verbindungselements 58a, zu dem Ventilgehäuse 12a (in Figuren nicht gezeigt). Insbesondere sind die Abschirmmittel dazu vorgesehen, einen elektrischen Kontakt zwischen den/dem jeweiligen Verbindungselement 56a, 58a und dem Ventilgehäuse 12a zu verhindern.

Alternativ ist denkbar, dass das Ventilgehäuse 12a eine Durchführung ausbildet, die zu einer Verbindung der Versorgungseinheit 50a mit der UV-Lichtquelle 38a vorgesehen ist. Alternativ oder zusätzlich ist denkbar, dass die Versorgungseinheit 50a dazu vorgesehen ist, elektrische Energie zu einer Versorgung der UV-Lichtquelle 38a aus dem Prozessmedium, insbesondere einer kinetischen und/oder thermischen Energie des Prozessmediums, und/oder aus einer Strömung in einer Pneumatik-Leitung des Stellventils 10a Energie zu einem Betrieb der UV-Lichtquelle 38a umzuwandeln (siehe Figur 2). Eine Versorgungseinheit 50a' zur Umwandlung von Energie aus dem Prozessmedium, insbesondere einer kinetischen und/oder thermischen Energie des Prozessmediums, in elektrische Energie ist in Figur 1 schematisch gezeigt. Die Versorgungseinheit 50a' umfasst zumindest ein Anregungselement 66a', welches zumindest teilweise innerhalb des Fluidkanals 32a angeordnet ist und zu einem Zusammenwirken mit dem Prozessmedium vorgesehen ist. Beispielsweise ist das Anregungselement 66a` als ein Thermoelement, als ein Wärmetauscher o.dgl. ausgebildet.

In Figur 1 ist eine weitere mögliche Anordnung einer UV-Lichtquelle 38a" der Sterilisierungseinheit 36a gezeigt, welche im Folgenden beschrieben wird. Diese ist alternativ oder zusätzlich zu der oben beschriebenen Anordnung der UV-Lichtquelle 38a denkbar. Das Ventilgehäuse 12a bildet eine Durchführung 68a" aus, die zu einer Verbindung der Versorgungseinheit 50a mit der UV-Lichtquelle 38a" vorgesehen ist. Die Durchführung 68a" ist im Bereich des Ventilsitzes 22a ausgebildet. Die Durchführung 68a" erstreckt sich von einer Außenseite des Ventilgehäuses 12a entlang der den Ventilsitz 22a ausbildenden Erhebung bis zur Drosselstelle 14a in den Fluidkanal 32a. Die Sterilisierungseinheit 36a umfasst ein Fensterelement 70a", das für UV-Strahlung der UV-Lichtquelle 38a" durchlässig ausgebildet ist, wobei das Fensterelement 70a" dazu vorgesehen ist, die UV-Lichtquelle 38a", insbesondere fluiddicht und/oder druckdicht, von dem Fluidkanal 32a zu trennen. Das Fensterelement 70a" ist im Wesentlichen vollständig in der Durchführung 68a" angeordnet. Das Fensterelement 70a" verschließt die Durchführung 68a" gegenüber dem Fluidkanal 32a für das Prozessmedium, insbesondere fluiddicht und/oder druckdicht. Die UV-Lichtquelle 38a" ist innerhalb der Durchführung 68a" angeordnet. Insbesondere ist die Durchführung 68a" als eine Ausnehmung in der Innenwand des Ventilgehäuses 12a ausgebildet. Die UV-Lichtquelle 38a" ist an dem Fensterelement 70a" angeordnet. Die UV-Lichtquelle 38a" ist derart angeordnet, dass die Hauptabstrahlrichtung 42a" der UV-Lichtquelle 38a" auf die Drosselstelle 14a gerichtet ist. Insbesondere ist die UV-Lichtquelle 38a" dazu vorgesehen, die UV-Strahlung zur Sterilisierung durch das Fensterelement 70a" in den Fluidkanal 32a auszugeben. Die UV-Lichtquelle 38a" ist in einem Nahbereich der Drosselstelle 14a angeordnet. Die UV-Lichtquelle 38a" ist derart angeordnet, dass die Hauptabstrahlrichtung 42a" der UV-Lichtquelle 38a" auf die Drosselstelle 14a gerichtet ist. Das Fensterelement 70a" ist für die von der UV-Lichtquelle 38a" zur Sterilisierung erzeugte Strahlung zumindest zu einem Großteil durchlässig ausgebildet. Das Fensterelement 70a" ist an dem Ventilgehäuse 12a befestigt, insbesondere innerhalb der Durchführung 68a". Das Fensterelement 70a" bedeckt die in der Durchführung 68a" angeordnete UV-Lichtquelle 38a" gegenüber dem Fluidkanal 32a im Wesentlichen vollständig, insbesondere fluiddicht und/oder druckdicht. Das Fensterelement 70a" ist derart ausgebildet und/oder angeordnet, dass eine dem Fluidkanal 32a zugewandte Außenfläche 72a" des Fensterelements 70a" im Wesentlichen bündig mit der Führungsfläche 47a des Ventilgehäuses 12a im Bereich der Drosselstelle 14a ausgebildet ist. Insbesondere ist das Fensterelement 70a", insbesondere die Außenfläche 72a" des Fensterelements 70a", zu einem Zusammenwirken mit dem Ventilglied 16a für ein Verschließen der Drosselstelle 14a vorgesehen. Insbesondere ist das Fensterelement 70a", insbesondere die Außenfläche 72a" des Fensterelements 70a", als Teil des Ventilsitzes 22a ausgebildet.

Es ist auch denkbar, dass das Ventilgehäuse 12a des Stellventils 10a an einer den Fluidkanal 32a begrenzenden Innenfläche eine Vertiefung und/oder eine Schweißnaht aufweist, wobei die/eine UV-Lichtquelle 38a, 38a" der Sterilisierungseinheit 36a in einem Nahbereich der Vertiefung und/oder der Schweißnaht angeordnet ist und/oder derart angeordnet ist, dass eine Hauptabstrahlrichtung 42a, 42a" der UV-Lichtquelle 38a, 38a" auf die Vertiefung und/oder die Schweißnaht gerichtet ist.

Es ist denkbar, dass die Sterilisierungseinheit 36a eine Mehrzahl von UV-Lichtquellen 38a, 38a" umfasst, die an einer Position gemäß den hier gezeigten Figuren innerhalb des Stellventils 10a, insbesondere an dem Ventilgehäuse 12a, angeordnet sind.

Alternativ oder zusätzlich ist denkbar, dass die UV-Lichtquelle 38a dazu vorgesehen ist, in zumindest einem Betriebszustand gepulst betrieben zu werden. Insbesondere ist die gepulst betriebene UV-Lichtquelle 38a von einer LED verschieden ausgebildet, beispielsweise als eine Quarz-Röhre mit Xenon-Gas, welche mittels eines elektrischen Pulses ansteuerbar ist.

In den Figuren 2 und 3 sind weitere Ausführungsbeispiele der Erfindung gezeigt. Die nachfolgenden Beschreibungen und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung des Ausführungsbeispiels, insbesondere der Figur 1, verwiesen werden kann. Zur Unterscheidung der Ausführungsbeispiele ist der Buchstabe a den Bezugszeichen des Ausführungsbeispiels in der Figur 1 nachgestellt. In den Ausführungsbeispielen der Figuren 2 und 3 ist der Buchstabe a jeweils durch den Buchstaben b und c ersetzt.

In Figur 2 ist ein als Hubventil ausgebildetes Stellventil 10b gezeigt. Das Stellventil 10b umfasst ein Ventilgehäuse 12b, das eine Drosselstelle 14b für ein Prozessmedium ausbildet, ein Ventilglied 16b, das einen Ventilkegel zum Verschließen der Drosselstelle 14b umfasst, eine Ventilstange 18b und eine Antriebseinheit 20b (in Figur 2 nur teilweise gezeigt), die dazu vorgesehen ist, das Ventilglied 16b über die Ventilstange 18b in einem Bereich der Drosselstelle 14b zu bewegen. Das Stellventil 10b umfasst eine Sterilisierungseinheit 36b zu einem Sterilisieren von Innenflächen 40b des Stellventils 10b, insbesondere des Ventilgehäuses 12b, und/oder des Prozessmediums, wobei die Sterilisierungseinheit 36b eine UV-Lichtquelle 38b umfasst. Das Ventilgehäuse 12b bildet einen Fluidkanal 32b aus. Das Ventilglied 16b ist für ein Verschließen des Stellventils 10b zu einem Zusammenwirken mit einem Ventilsitz 22b des Stellventils 10b vorgesehen. Das in der Figur 2 dargestellte Stellventil 10b weist neben der Ausgestaltung als Hubventil eine zumindest im Wesentlichen analoge Ausgestaltung zu dem in der Beschreibung der Figur 1 beschriebenen Stellventil 10a auf, so dass bezüglich einer Ausgestaltung des in der Figur 2 dargestellten Stellventils 10b zumindest im Wesentlichen auf die Beschreibung der Figur 1 verwiesen werden kann. Im Unterschied zu dem in Figur 1 gezeigten Stellventil 10a umfasst das Ventilgehäuse 12b des in der Figur 2 gezeigten Stellventils 10b eine Durchführung 68b im Bereich einer Verbindungsstelle 24b des Stellventils 10b, die insbesondere eine Eintrittsöffnung 28b des Ventilgehäuses 12b ausbildet. Alternativ oder zusätzlich ist eine Anordnung der Durchführung 68b bzw. der UV-Lichtquelle an einer anderen Verbindungsstelle 26b, 34b des Stellventils 10b denkbar. Insbesondere ist die Durchführung 68b als eine Ausnehmung in der Innenwand des Ventilgehäuses 12b ausgebildet. Die Durchführung 68b ist durch eine Innenfläche 44b des Ventilgehäuses 12b begrenzt, wobei insbesondere die UV-Lichtquelle 38b an der Innenfläche 44b angeordnet ist. Die Sterilisierungseinheit 36b umfasst ein Fensterelement 70b, das für UV-Strahlung der UV-Lichtquelle 38b durchlässig ausgebildet ist, wobei das Fensterelement 70b dazu vorgesehen ist, die UV-Lichtquelle 38b, insbesondere fluiddicht und/oder druckdicht, von dem Fluidkanal 32b zu trennen. Die Durchführung 68b erstreckt sich von einer Außenseite des Ventilgehäuses 12b an der Verbindungsstelle 24b bis in den Fluidkanal 32b. Die Verbindungsstelle 24b ist als ein Rohrflansch ausgebildet und weist insbesondere einen größeren Außendurchmesser auf wie eine zur Verbindungsstelle 24b benachbarte Wandung des Ventilgehäuses 12b. Das Fensterelement 70b ist im Wesentlichen vollständig innerhalb der Durchführung 68b angeordnet. Das Fensterelement 70b verschließt die Durchführung 68b gegenüber dem Fluidkanal 32b für das Prozessmedium, insbesondere fluiddicht und/oder druckdicht. Die UV-Lichtquelle 38b ist innerhalb der Durchführung 68b angeordnet. Die UV-Lichtquelle 38b ist an dem Fensterelement 70b angeordnet. Die UV-Lichtquelle 38b ist derart angeordnet, dass eine Hauptabstrahlrichtung 42b der UV-Lichtquelle 38b in Richtung des Fluidkanals 32b ausgerichtet ist. Insbesondere ist die UV-Lichtquelle 38b dazu vorgesehen, die UV-Strahlung zur Sterilisierung durch das Fensterelement 70b in den Fluidkanal 32b auszugeben. Die UV-Lichtquelle 38b ist in einem Nahbereich der Verbindungsstelle 24b angeordnet. Die Durchführung 68b erstreckt sich über eine gesamte Wandstärke des Ventilgehäuses 12b im Bereich der Verbindungsstelle 24b. Das Fensterelement 70b ist an dem Ventilgehäuse 12b befestigt, insbesondere innerhalb der Durchführung 68b. Das Fensterelement 70b bedeckt die in der Durchführung 68b angeordnete UV-Lichtquelle 38b gegenüber dem Fluidkanal 32b im Wesentlichen vollständig, insbesondere fluiddicht und/oder druckdicht. Das Fensterelement 70b ist derart ausgebildet und/oder angeordnet, dass eine dem Fluidkanal 32b zugewandte Außenfläche 72b des Fensterelements 70b im Wesentlichen bündig mit einer Führungsfläche 47b des Ventilgehäuses 12b, die insbesondere den Fluidkanal 32b innerhalb des Ventilgehäuses 12b begrenzt, im Bereich der Verbindungsstelle 24b ausgebildet ist. Analog zu einer Anordnung der UV-Lichtquelle 38b, des Fensterelements 70b und der Durchführung 68b des Stellventils 10b sind auch andere Ausgestaltungen eines Stellventils denkbar, wobei beispielsweise eine UV-Lichtquelle, ein Fensterelement und eine Durchführung an einer anderen Stelle des Ventilgehäuses 12b angeordnet sind, beispielsweise an der anderen Verbindungsstelle 34a, im Bereich der an die Drosselstelle 14b grenzenden Kammern des Fluidkanals 32b, vorzugsweise seitlich an dem Ventilgehäuse 12b, o.dgl.

Die Sterilisierungseinheit 36b umfasst eine Versorgungseinheit 50b zu einer elektrischen Versorgung der UV-Lichtquelle 38b. Das Stellventil 10b umfasst eine Pneumatik-Leitung 74b zu einem Betrieb der Antriebseinheit 20b und/oder eines Stellungsreglers des Stellventils 10b. Die Versorgungseinheit 50b ist dazu vorgesehen, aus einer Strömung in der Pneumatik-Leitung 74b Energie zu einem Betrieb der UV-Lichtquelle 38b umzuwandeln. Die Versorgungseinheit 50b umfasst ein Anregungselement 76b, welches zumindest teilweise in oder an der Pneumatik-Leitung 74b angeordnet ist, und einen Generator 78b zur Umwandlung einer Bewegung des Anregungselements 76b in elektrische Energie. Alternativ ist denkbar, dass das Anregungselement 76b dazu vorgesehen ist, einen Druck in der Pneumatik-Leitung 74b in elektrische Energie umzuwandeln, wobei das Anregungselement 76b beispielsweise als piezoelektrisches Element ausgebildet ist. Die Versorgungseinheit 50b umfasst ein Übertragungselement 64b, welches dazu vorgesehen ist, den Generator 78b und die UV-Lichtquelle 38b elektrisch zu verbinden. Das Übertragungselement 64b ist durch die Durchführung 68b bis an die UV-Lichtquelle 38b geführt. Alternativ sind auch andere Ausgestaltungen der Versorgungseinheit 50b zu einer Umwandlung von Energie aus der Pneumatik-Leitung 74b in elektrische Energie denkbar.

Alternativ oder zusätzlich ist denkbar, dass die UV-Lichtquelle 38b in einem Bereich des Fluidkanals 32b mit einer zu erwartenden minimalen lokalen Strömungsgeschwindigkeit des Prozessmediums angeordnet ist und/oder derart angeordnet ist, dass die Hauptabstrahlrichtung 42b der UV-Lichtquelle 38b auf den Bereich des Fluidkanals 32b mit einer zu erwartenden minimalen lokalen Strömungsgeschwindigkeit des Prozessmediums gerichtet ist.

In Figur 2 sind weitere mögliche Anordnungen von UV-Lichtquellen 38b` der Sterilisierungseinheit 36b gezeigt. Es ist denkbar, dass eine UV-Lichtquelle 38b` der Sterilisierungseinheit 36b in eine Prozessrichtung 80b betrachtet hinter der Drosselstelle 14b an einem Ventilsitz 22b des Stellventils 10b angeordnet sind. Insbesondere ist der Ventilsitz 22b des Stellventils 10b von dem Ventilgehäuse 12b abnehmbar ausgebildet. Es ist denkbar, dass die UV-Lichtquelle 38b` an dem Ventilsitz 22b angeordnet ist und mit dem Ventilsitz 22b oder unabhängig von dem Ventilsitz 22b austauschbar ausgebildet ist. Insbesondere ist denkbar, dass mehrere UV-Lichtquellen 38b` um eine Mittelachse 84b der Drosselstelle 14b bzw. des Ventilsitzes 22b verteilt angeordnet sind, insbesondere um ein durchströmendes Prozessmedium zu sterilisieren. Bevorzugt sind die UV-Lichtquellen 38b` gleichmäßig um die Mittelachse 84b der Drosselstelle 14b bzw. des Ventilsitzes 22b verteilt angeordnet. Alternativ oder zusätzlich ist denkbar, dass eine UV-Lichtquelle 38b" der Sterilisierungseinheit 36b vor der Drosselstelle 14b angeordnet ist, insbesondere an einem Boden des Ventilgehäuses 12b. Die UV-Lichtquelle 38b" ist zumindest teilweise innerhalb einer Ausnehmung 86b" des Ventilgehäuses 12b angeordnet. Die UV-Lichtquelle 38b" ist derart angeordnet, dass eine Hauptabstrahlrichtung 42b" der UV-Lichtquelle 38b" in Richtung der Drosselstelle 14b zeigt.

Die in der Figur 2 gezeigten Positionen zur Anordnung von UV-Lichtquellen 38b. 38b`, 38b" sind jeweils auch in anderen Ausgestaltungen des Ventilgehäuses 12b an der jeweiligen Stelle denkbar, beispielsweise mit einer Durchführung 68b, einer Ausnehmung 86b" o.dgl. und/oder mit oder ohne ein Fensterelement 70b.

In Figur 3 ist ein als Hubventil ausgebildetes Stellventil 10c gezeigt. Das Stellventil 10c umfasst ein Ventilgehäuse 12c, das eine Drosselstelle 14c für ein Prozessmedium ausbildet, ein Ventilglied 16c, das einen Ventilkegel zum Verschließen der Drosselstelle 14c umfasst, eine Ventilstange 18c und eine Antriebseinheit 20c (in Figur 3 nur teilweise gezeigt), die dazu vorgesehen ist, das Ventilglied 16c über die Ventilstange 18c in einem Bereich der Drosselstelle 14c zu bewegen. Das Stellventil 10c umfasst eine Sterilisierungseinheit 36c zu einem Sterilisieren von Innenflächen 40c des Stellventils 10c, insbesondere des Ventilgehäuses 12c, und/oder des Prozessmediums, wobei die Sterilisierungseinheit 36c eine UV-Lichtquelle 38c umfasst. Das Ventilgehäuse 12c bildet einen Fluidkanal 32c aus. Das in der Figur 3 dargestellte Stellventil 10c weist eine zumindest im Wesentlichen analoge Ausgestaltung zu dem in der Beschreibung der Figur 2 beschriebenen Stellventil 10b auf, so dass bezüglich einer Ausgestaltung des in der Figur 3 dargestellten Stellventils 10c zumindest im Wesentlichen auf die Beschreibung der Figur 2 verwiesen werden kann. Im Unterschied zu dem in Figur 2 gezeigten Stellventil 10b umfasst das Ventilgehäuse 12c des in der Figur 3 gezeigten Stellventils 10c eine Versorgungs-Verbindungsstelle 88c zu einer Befestigung eines Versorgungsverbinders 90c des Stellventils 10c, der zu einer Durchführung eines Übertragungselements 64c einer Versorgungseinheit 50c der Sterilisierungseinheit 36c in den Fluidkanal 32c vorgesehen ist. Die Versorgungseinheit 50c ist zu einer elektrischen Versorgung der UV-Lichtquelle 38c vorgesehen. Die Versorgungs-Verbindungsstelle 88c ist insbesondere als ein Flansch, insbesondere als ein üblicher Rohrflansch, ausgebildet. Die Versorgungseinheit 50c ist dazu vorgesehen, die UV-Lichtquelle 38c durch die Versorgungs-Verbindungsstelle 88c hindurch mit elektrischer Energie zu versorgen. Der Versorgungsverbinder 90c ist zu einer Befestigung an der Versorgungs-Verbindungsstelle 88c vorgesehen. Der Versorgungsverbinder 90c ist korrespondierend zu der Versorgungs-Verbindungsstelle 88c ausgebildet. Der Versorgungsverbinder 90c ist insbesondere als ein Flanschaufsatz ausgebildet und dazu vorgesehen, das Ventilgehäuse 12c im Bereich der Versorgungs-Verbindungsstelle 88c insbesondere für das Prozessmedium fluiddicht und/oder druckdicht zu verschließen. Der Versorgungsverbinder 90c ist vorzugsweise dazu vorgesehen, das Übertragungselement 64c der Versorgungseinheit 50c von außen durch die Versorgungs-Verbindungsstelle 88c in den Fluidkanal 32c zu führen. Bevorzugt ist der Versorgungsverbinder 90c dazu vorgesehen, das Übertragungselement 64c gegenüber dem Ventilgehäuse 12c elektrisch zu isolieren. Der Versorgungsverbinder 90c verdeckt die Versorgungs-Verbindungsstelle 88c im Wesentlichen vollständig. Der Versorgungsverbinder 90c ist insbesondere dazu vorgesehen, über Befestigungsmittel an der Versorgungs-Verbindungsstelle 88c bzw. dem Ventilgehäuse 12c befestigt zu werden. Das Ventilgehäuse 12c bildet die Versorgungs-Verbindungsstelle 88c aus. Insbesondere sind das Ventilgehäuse 12c und die Versorgungs-Verbindungsstelle 88c einteilig ausgebildet. Es ist denkbar, dass das Stellventil 10c ein Abdeckteil umfasst, dass dazu vorgesehen ist, die Versorgungs-Verbindungsstelle 88c in Abwesenheit einer UV-Lichtquelle 38c bzw. des Versorgungsverbinders 90c abzudecken, insbesondere fluiddicht und/oder druckdicht zu verschließen. Die UV-Lichtquelle 38c ist an einem Trägerelement 92c der Sterilisierungseinheit 36c angeordnet, welches an dem Versorgungsverbinder 90c abgestützt ist. Alternativ ist denkbar, dass das Trägerelement 92c an dem Ventilgehäuse 12c angeordnet, insbesondere befestigt, ist. Die UV-Lichtquelle 38c ist innerhalb des Fluidkanals 32c angeordnet. Das Trägerelement 92c erstreckt sich von der Versorgungs-Verbindungsstelle 88c bzw. dem Versorgungsverbinder 90c in den Fluidkanal 32c hinein. Das Übertragungselement 64c ist durch den Versorgungsverbinder 90c in den Fluidkanal 32c geführt und verläuft entlang oder innerhalb des Trägerelements 92c bis zur UV-Lichtquelle 38c. Insbesondere ist denkbar, dass das Trägerelement 92c dazu vorgesehen ist, das Übertragungselement 64c vor dem Prozessmedium zu schützen, insbesondere gegenüber dem Fluidkanal 32c, insbesondere fluiddicht und/oder druckdicht, zu verschließen. Die UV-Lichtquelle 38c ist derart angeordnet, dass eine Hauptabstrahlrichtung 42c der UV-Lichtquelle 38c in Richtung der Drosselstelle 14c zeigt. Es sind aber auch andere Ausgestaltungen bzw. Ausrichtungen der UV-Lichtquelle 38c an dem Trägerelement 92c denkbar, wobei die UV-Lichtquelle 38c beispielsweise auf eine Innenfläche 40c des Ventilgehäuses 12c, eine Vertiefung des Stellventils 10c, insbesondere des Ventilgehäuses 12c, eine Schweißnaht des Stellventils 10c, insbesondere des Ventilgehäuses 12c, o.dgl. gerichtet ist.

Alternativ ist denkbar, dass die UV-Lichtquelle 38c an einer anderen Stelle innerhalb des Ventilgehäuses 12c angeordnet ist, insbesondere zusammen mit dem Trägerelement 92c oder direkt an einer Innenfläche 40c des Ventilgehäuses 12c (vgl. Figuren 1 und 2), und/oder dass die Versorgungs-Verbindungsstelle 88c an einer anderen Stelle des Ventilgehäuses 12c ausgebildet ist.

Besonders bevorzugt umfasst die Sterilisierungseinheit 36c ein Fensterelement 70c, das für UV-Strahlung der UV-Lichtquelle 38c durchlässig ausgebildet ist, wobei das Fensterelement 70c dazu vorgesehen ist, die zumindest eine UV-Lichtquelle 38c, insbesondere fluiddicht und/oder druckdicht, von dem Fluidkanal 32c bzw. dem Prozessmedium zu trennen. Insbesondere umschließt das Fensterelement 70c die an dem Trägerelement 92c angeordnete UV-Lichtquelle 38c im Wesentlichen vollständig. Es ist denkbar, dass das Fensterelement 70c dazu vorgesehen ist, das Übertragungselement 64c und/oder das Trägerelement 92c vor dem Prozessmedium zu schützen bzw. zu umschließen.

**Bezugszeichen**

| | | | |
|---|---|---|---|
| 10 | Stellventil | 48 | Abstand |
| 12 | Ventilgehäuse | 50 | Versorgungseinheit |
| 14 | Drosselstelle | 54 | Wandung |
| 16 | Ventilglied | 56 | Verbindungselement |
| 18 | Ventilstange | 58 | Verbindungselement |
| 20 | Antriebseinheit | 60 | Energiespeicher |
| 22 | Ventilsitz | 64 | Übertragungselement |
| 24 | Verbindungsstelle | 66 | Anregungselement |
| 26 | Verbindungsstelle | 68 | Durchführung |
| 28 | Eintrittsöffnung | 70 | Fensterelement |
| 30 | Austrittsöffnung | 72 | Außenfläche |
| 32 | Fluidkanal | 74 | Pneumatik-Leitung |
| 34 | Verbindungsstelle | 76 | Anregungselement |
| 36 | Sterilisierungseinheit | 78 | Generator |
| 38 | UV-Lichtquelle | 80 | Prozessrichtung |
| 40 | Innenfläche | 84 | Mittelachse |
| 42 | Hauptabstrahlrichtung | 86 | Ausnehmung |
| 44 | Innenfläche | 88 | Versorgungs-Verbindungsstelle |
| 46 | Mittelachse | 90 | Versorgungsverbinders |
| 47 | Führungsfläche | 92 | Trägerelement |

## Patentansprüche

1. Stellventil für eine Prozessanlage, insbesondere mit hohen Reinlichkeitsanforderungen, beispielsweise in der Lebensmittel- und/oder der Pharmaindustrie, mit einem Ventilgehäuse (12a; 12b; 12c), das zumindest eine Drosselstelle (14a; 14b; 14c) für ein Prozessmedium ausbildet, mit einem Ventilglied (16a; 16b; 16c), mit einer Ventilstange (18a; 18b; 18c) und mit einer Antriebseinheit (20a; 20b; 20c), die dazu vorgesehen ist, das Ventilglied (16a; 16b; 16c) über die Ventilstange (18a; 18b; 18c) in einem Bereich der Drosselstelle (14a; 14b; 14c) zu bewegen, **gekennzeichnet durch** eine Sterilisierungseinheit (36a; 36b; 36c) zu einem Sterilisieren von Innenflächen (40a; 40b; 40c) des Stellventils (10a; 10b, 10c), insbesondere des Ventilgehäuses (12a; 12b; 12c), und/oder des Prozessmediums, wobei die Sterilisierungseinheit (36a; 36b; 36c) zumindest eine UV-Lichtquelle (38a, 38a"; 38b; 38b', 38b"; 38c) umfasst.

2. Stellventil nach Anspruch 1, **dadurch gekennzeichnet, dass** die zumindest eine UV-Lichtquelle (38a, 38a"; 38b; 38b', 38b"; 38c) als eine LED ausgebildet ist und insbesondere dazu vorgesehen ist, Strahlung im UVc-Bereich des elektromagnetischen Spektrums zu erzeugen.

3. Sterilisierungsvorrichtung nach Anspruch 1 oder 2, **dadurch gekennzeichnet, dass** die zumindest eine UV-Lichtquelle (38a, 38a"; 38b; 38b', 38b"; 38c) dazu vorgesehen ist, in zumindest einem Betriebszustand gepulst betrieben zu werden.

4. Stellventil nach einem der vorhergehenden Ansprüche, **dadurch gekennzeichnet, dass** das Ventilgehäuse (12a; 12b; 12c) zumindest einen Fluidkanal (32a; 32b; 32c) für das Prozessmedium ausbildet, der zumindest teilweise von der Drosselstelle (14a; 14b; 14c) begrenzt ist, wobei das Ventilgehäuse (12a; 12b; 12c) zumindest eine dem Fluidkanal (32a; 32b; 32c) zugewandte Innenfläche (44a; 44b; 44c) aufweist, wobei die zumindest eine UV-Lichtquelle (38a, 38a"; 38b; 38b', 38b"; 38c) an der Innenfläche (44a; 44b; 44c) des Ventilgehäuses (12a; 12b; 12c) angeordnet ist.

5. Stellventil nach einem der vorhergehenden Ansprüche, **dadurch gekennzeichnet, dass** das Ventilgehäuse (12a; 12b; 12c) zumindest einen Fluidkanal (32a; 32b; 32c) für das Prozessmedium ausbildet, der zumindest teilweise von der Drosselstelle (14a; 14b; 14c) begrenzt ist, wobei die zumindest eine UV-Lichtquelle (38a; 38b', 38b"; 38c) zumindest teilweise innerhalb des Fluidkanals (32a; 32b; 32c) angeordnet ist.

6. Stellventil nach Anspruch 4, **dadurch gekennzeichnet, dass** das Ventilgehäuse (12a; 12b) an einer den Fluidkanal (32a; 32b) begrenzenden Innenwand eine Ausnehmung (68a"; 68b, 86b") begrenzt, wobei die zumindest eine UV-Lichtquelle (38a"; 38b, 38b") zumindest teilweise, insbesondere im Wesentlichen vollständig, innerhalb der Ausnehmung (68a"; 68b, 86b") angeordnet ist.

7. Stellventil nach einem der vorhergehenden Ansprüche, **dadurch gekennzeichnet, dass** die Sterilisierungseinheit (36a; 36b; 36c) ein Fensterelement (70a"; 70b; 70c) umfasst, das für UV-Strahlung der zumindest einen UV-Lichtquelle (38a"; 38b; 38c) durchlässig ausgebildet ist, wobei das Fensterelement (70a"; 70b; 70c) dazu vorgesehen ist, die zumindest eine UV-Lichtquelle (38a"; 38b; 38c), insbesondere fluiddicht und/oder druckdicht, von dem Fluidkanal (32a; 32b; 32c) zu trennen.

8. Stellventil nach einem der vorhergehenden Ansprüche, **dadurch gekennzeichnet, dass** die zumindest eine UV-Lichtquelle (38a";38b`; 38c) in einem Nahbereich der Drosselstelle (14a; 14b; 14c) angeordnet ist und/oder derart angeordnet ist, dass eine Hauptabstrahlrichtung (42a"; 42c) der UV-Lichtquelle (38a"; 238c) auf die Drosselstelle (14a; 14b; 14c) und/oder auf den Nahbereich der Drosselstelle (14a; 14b; 14c) gerichtet ist.

9. Stellventil nach einem der vorhergehenden Ansprüche, **dadurch gekennzeichnet, dass** die Sterilisierungseinheit (36a; 36b) eine Versorgungseinheit (50a; 50b) zu einer elektrischen Versorgung der zumindest einen UV-Lichtquelle (38a, 38a"; 38b; 38b`, 38b") umfasst, wobei das Ventilgehäuse (12a; 12b) eine Durchführung (68a"; 68b) ausbildet, die zu einer Verbindung der Versorgungseinheit (50a; 50b) mit der UV-Lichtquelle (38a, 38a"; 38b; 38b', 38b") vorgesehen ist.

10. Stellventil nach einem der vorhergehenden Ansprüche, **dadurch gekennzeichnet, dass** die Sterilisierungseinheit (36a) eine Versorgungseinheit (50a') zu einer elektrischen Versorgung der zumindest einen UV-Lichtquelle (38a, 38a") umfasst, wobei die Versorgungseinheit (50a') dazu vorgesehen ist, elektrische Energie zu einer Versorgung der UV-Lichtquelle (38a, 38a") aus dem Prozessmedium, insbesondere einer kinetischen und/oder thermischen Energie des Prozessmediums, umzuwandeln.

11. Stellventil nach einem der vorhergehenden Ansprüche, **gekennzeichnet durch** zumindest eine Pneumatik-Leitung (74b) zu einem Betrieb der Antriebseinheit (20b) und/oder eines Stellungsreglers des Stellventils (10b), wobei die Sterilisierungseinheit (36a; 36b) eine Versorgungseinheit (50a; 50b) zu einer elektrischen Versorgung der zumindest einen UV-Lichtquelle (38b; 38b`, 38b") umfasst, wobei die Versorgungseinheit (50b) dazu vorgesehen ist, aus einer Strömung in der Pneumatik-Leitung (74b) Energie zu einem Betrieb der UV-Lichtquelle (38b; 38b`, 38b") umzuwandeln.

12. Stellventil nach einem der vorhergehenden Ansprüche, **dadurch gekennzeichnet, dass** die Sterilisierungseinheit (36a) eine Versorgungseinheit (50a) zu einer elektrischen Versorgung der zumindest einen UV-Lichtquelle (38a, 38a") umfasst, wobei die Versorgungseinheit (50a) dazu vorgesehen ist, die zumindest eine UV-Lichtquelle (38a, 38a") kabellos, insbesondere durch zumindest eine Wandung (54a) des Ventilgehäuses (12a), mit elektrischer Energie zu versorgen.

13. Stellventil nach einem der vorhergehenden Ansprüche, **dadurch gekennzeichnet, dass** das Ventilgehäuse (12a; 12b; 12c) zumindest einen Fluidkanal (32a; 32b; 32c) für das Prozessmedium ausbildet, der zumindest teilweise von der Drosselstelle (14a; 14b; 14c) begrenzt ist, wobei die zumindest eine UV-Lichtquelle (38a, 38a"; 38b; 38b', 38b"; 38c) in einem Bereich des Fluidkanals (32a; 32b; 32c) mit einer zu erwartenden minimalen lokalen Strömungsgeschwindigkeit des Prozessmediums angeordnet ist und/oder derart angeordnet ist, dass eine Hauptabstrahlrichtung (42a, 42a"; 42b, 42b"; 42c) der UV-Lichtquelle (38a, 38a"; 38b; 38b', 38b"; 38c) auf den Bereich des Fluidkanals (32a; 32b; 32c) mit einer zu erwartenden minimalen lokalen Strömungsgeschwindigkeit des Prozessmediums gerichtet ist.

14. Stellventil nach einem der vorhergehenden Ansprüche, **dadurch gekennzeichnet, dass** das Ventilgehäuse (12a; 12b; 12c) zumindest einen Fluidkanal (32a; 32b; 32c) für das Prozessmedium ausbildet, der zumindest teilweise von der Drosselstelle (14a; 14b; 14c) begrenzt ist, wobei das Ventilgehäuse (12a; 12b; 12c) an einer den Fluidkanal (32a; 32b; 32c) begrenzenden Innenfläche (40a; 40b; 40c) eine Vertiefung und/oder eine Schweißnaht aufweist, wobei die zumindest eine UV-Lichtquelle (38a, 38a"; 38b; 38b', 38b"; 38c) in einem Nahbereich der Vertiefung und/oder der Schweißnaht angeordnet ist und/oder derart angeordnet ist, dass eine Hauptabstrahlrichtung (42a, 42a"; 42b, 42b"; 42c) der UV-Lichtquelle (38a, 38a"; 38b; 38b', 38b"; 38c) auf die Vertiefung und/oder die Schweißnaht gerichtet ist.

15. Stellventil nach einem der vorhergehenden Ansprüche, **dadurch gekennzeichnet, dass** das Ventilgehäuse (12a; 12b) eine Verbindungsstelle (24a, 26a, 34a; 24b, 26b, 34b), insbesondere einen Flansch und/oder eine Dichtungskante, umfasst, wobei die zumindest eine UV-Lichtquelle (38a, 38a"; 38b; 38b', 38b") in einem Nahbereich der Verbindungsstelle (24a, 26a, 34a; 24b, 26b, 34b) angeordnet ist und/oder derart angeordnet ist, dass eine Hauptabstrahlrichtung (42a, 42a"; 42b, 42b") der UV-Lichtquelle (38a, 38a"; 38b; 38b', 38b") auf die Verbindungsstelle (24a, 26a, 34a; 24b, 26b, 34b) und/oder auf den Nahbereich der Verbindungsstelle (24a, 26a, 34a; 24b, 26b, 34b) gerichtet ist.

16. Stellventil nach einem der vorhergehenden Ansprüche, **dadurch gekennzeichnet, dass** die Sterilisierungseinheit (36c) zumindest eine Versorgungseinheit (50c) zu einer elektrischen Versorgung der zumindest einen UV-Lichtquelle (38c) umfasst und das Ventilgehäuse (12c) eine Versorgungs-Verbindungsstelle (88c), insbesondere einen Flansch, umfasst, wobei die Versorgungseinheit (50c) dazu vorgesehen ist, die UV-Lichtquelle (38c) durch die Versorgungs-Verbindungsstelle (88c) hindurch mit elektrischer Energie zu versorgen.
